(19) Europäisches Patentamt
European Patent Office
Office européen des brevets

(11) EP 3 922 269 A1

(12) EUROPEAN PATENT APPLICATION

(43) Date of publication:
**15.12.2021 Bulletin 2021/50**

(21) Application number: **21189671.7**

(22) Date of filing: **16.03.2017**

(51) Int Cl.:
***A61K 47/69*** (2017.01) ***A61K 47/60*** (2017.01)
***C07K 14/605*** (2006.01) ***A61K 38/00*** (2006.01)
***A61P 3/10*** (2006.01) ***A61K 38/26*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2016 US 201662309330 P**
**01.11.2016 US 201662416058 P**

(62) Document number(s) of the earlier application(s) in accordance with Art. 76 EPC:
**17767565.9 / 3 429 608**

(71) Applicant: **Prolynx LLC**
**San Francisco, CA 94158 (US)**

(72) Inventors:
- **SCHNEIDER, Eric L.**
  **San Francisco, 94158 (US)**
- **HEARN, Brian**
  **San Francisco, 94158 (US)**
- **HENISE, Jeffrey C.**
  **San Francisco, 94158 (US)**
- **ASHLEY, Gary W.**
  **San Francisco, 94158 (US)**
- **SANTI, Daniel V.**
  **San Francisco, 94158 (US)**

(74) Representative: **V.O.**
**P.O. Box 87930**
**2508 DH Den Haag (NL)**

Remarks:
This application was filed on 04.08.2021 as a divisional application to the application mentioned under INID code 62.

# (54) EXTENDED RELEASE CONJUGATES OF EXENATIDE ANALOGS

(57) Extended-release conjugates of stabilized GLP-1 agonists balance agonist stability with release rates to provide long lasting administration to treat diabetes and related conditions on once-a-month or less frequent dosing schedules.

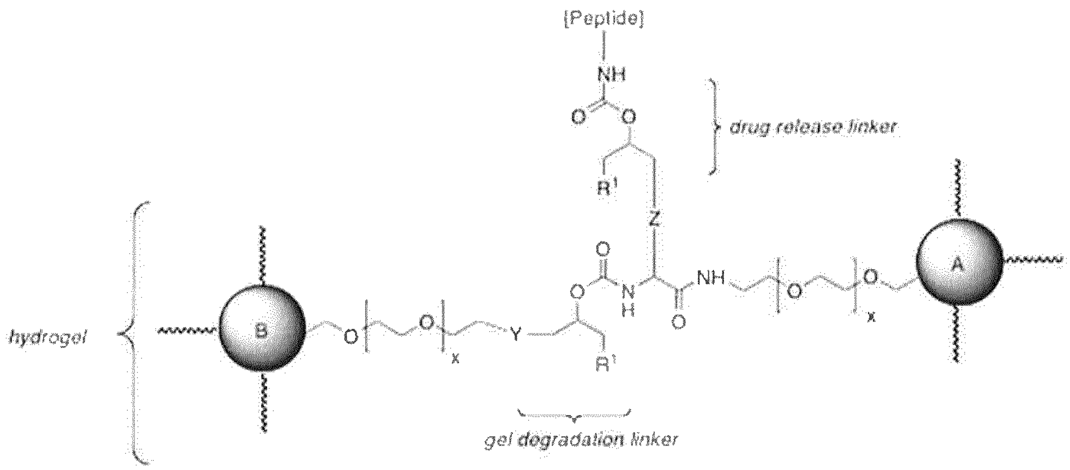


Y,Z = connecting functionality

Figure 4

EP 3 922 269 A1

## Description

### Technical Field

The invention is in the field of drug-delivery and sustained-release formulations. More particularly, it concerns sustained-release compositions that deliver stabilized forms of GLP-1 agonists over periods of one month or more.

### Background Art

Exenatide is a 39-amino acid peptide that is a potent agonist of the GLP-1 receptor, making it an insulin secretagogue with glucoregulatory effects. It is widely used in the treatment of type 2 diabetes as the free peptide, marketed as Byetta® (Astra-Zeneca), the peptide is injected twice-daily due to the short in vivo half-life of 2.5 hours. It is highly desirable to extend the half-life of exenatide and related GLP-1 agonist peptides so as to improve their efficacy, decrease side effects, and ease the treatment burden on patients.

Peptide half-life is traditionally extended by one or a combination of several methods: (i) chemical modification of the peptide to slow metabolism; (ii) encapsulation to provide a slow-release depot formulation; and (iii) conjugation with a macromolecule to slow clearance. See, for example, Cai, et al., Drug Design, Development, and Therapy (2013) 7:963-970.

Chemical modifications of the peptide to increase half-life have resulted in once-daily GLP-1 agonists, for example lixisenatide (Lyxumia®) and liraglutide (Victoza®). Encapsulation of the peptide into PLGA (poly lactic-coglycolic acid) microparticles has been used to produce a slow-release formulation, marketed as Bydureon® (Astra-Zeneca), that allows for once-weekly subcutaneous injection. Attempts to extend the duration of Bydureon to once-monthly administrations using triglyceride formulations have not yet proven successful. Conjugation of GLP-1 peptide agonists with Fc antibody domains or with random-sequence polypeptides (XTEN) has been able to extend the half-life only up to 5-6 days.

It is convenient to consider the plasma half-life, the time required to lose one half of the drug from the system, in understanding dosing requirements. Dosing frequency is determined by the need to maintain drug levels at or above a certain efficacious level. If dosing is to occur once every half-life, then the dose must be such as to give an initial drug level 2x the efficacious level; similarly, if dosing to occur once every 2 half-lives, then the dose must be such as to give an initial drug level 4x the efficacious level. In theory, dosing could be made as infrequent as desired regardless of the half-life simply by increasing the amount of drug given per dose. Many drugs show toxicities that are related to high initial concentrations, however, placing a practical limitation on dosing frequency as a function of half-life. Dosing intervals of approximately 1-2 half-lives are typical. While several preparations of GLP-1 agonist peptides having suitable effective half-lives for once-monthly administration have been disclosed, these suffer several disadvantages. Encapsulating microspheres and phase-transition lipid formulations may suffer from initial burst-phase release of the peptide, exposing the patient to an initial undesirably high drug level. Implantable pumps require surgery for implantation and removal.

Initial bursts of drug release can be avoided through the use of covalently linked conjugates. While soluble, circulating conjugates typically do not have a sufficient half-life themselves to support once-monthly drug administration (the maximal half-life of poly(ethylene glycol) in humans is about 1 week), insoluble conjugate implants such as hydrogels are suitable. Compositions wherein drugs such as exenatide are covalently linked to various matrices through linkers having controllable rates of drug release have been previously disclosed, for example in U.S. Patents 8,680,315; U.S. 8,754,190; U.S. 8,703,907, including insoluble matrices as disclosed in U.S. 8,946,405 and from hydrogels as disclosed in US2014/0288190 ('190). In one embodiment in the '190 publication, exenatide is linked to a hydrogel matrix wherein upon injection into the subcutaneous space, the hydrogel provides a depot from which exenatide is released by beta-eliminative cleavage of the linker to provide a long-acting source of the drug. Such hydrogels are by definition composed primarily of water, and the exenatide is therefore exposed to an aqueous environment for the duration of the depot. While this aqueous environment is considered advantageous for maintaining the complex structure of proteins, certain peptide sequences may suffer instabilities under such long-term conditions.

It has been found that the rate of degradation of exenatide in hydrogels under physiological conditions makes once-monthly or even less frequent administration of such formulations impractical. Exenatide has been reported to show chemical instabilities under stressed conditions (pH 7.9, 40°C for 6 days) resulting from oxidation at M14 and W25 and from side-chain amide hydrolysis at Q13 and N28 (US patent application 2006/0194719; Zealand). Detailed kinetics of the instability were not disclosed. While a detailed model for deamidation of Asn residues in peptides has been described (Geiger & Clarke, J. Biol. Chem. (1987) 262:785-794), the rate of deamidation of peptides and proteins under physiological conditions is known to be highly variable (Robinson & Robinson, Proc Natl Acad Sci (2010) 98:12409-12413). Exenatide itself is sufficiently stable for twice-daily administration (Byetta®), and for once-weekly administration via a PLGA implant (Bydureon®).

Stabilized forms of exenatide have been disclosed. For example, PCT application WO2008/116294 (Matregen) discloses stabilized exenatide analogs modified at three positions: Q13, M14 and N28. While multiple amino acid substitutions in the exenatide sequence can be tolerated, it has now been found that replacement of N28 by a more stable residue is sufficient for effective stabilization of the peptide at pH 7.4, 37 °C. Several products of exenatide deamidation, for example N28D and N28-isoD, have been disclosed in the above-cited US patent application 2006/0194719 (Zealand) and found to maintain potency for GLP-1 receptor activation. These products form an interconverting system, however, and are unstable towards equilibration to the original mixture of products.

Detailed investigations of the fate of exenatide in aqueous buffers at pH 7.4, 37°C, (*i.e.*, physiological pH and temperature) have shown that it undergoes deamidation at Asn28 (N28) with a half-life of between 8 and 14 days, depending on the buffer. A hydrogel conjugate designed for once-monthly administration would thus be releasing primarily degradation products of exenatide after 8-14 days. It is thus essential for the stabilized GLP-1 agonist to have a sufficient resistance toward chemical degradation to minimize the amount of degraded forms released by the end of the administration period. This is achieved when the GLP-1 agonist forms less than 10% degradation products after one month at pH 7.4, 37°C, preferably less than 5% degradation products after one month at pH 7.4, 37°C.

Disclosure of the Invention

The present invention is directed to conjugates that provide extended release of stabilized GLP-1 agonist peptides that support once-monthly or even less frequent administration of these peptides and are useful in the treatment of metabolic diseases and conditions such as metabolic syndrome, diabetes and obesity. The conjugates combine the extended stability of the GLP-1 agonist with the controlled release time provided by a suitable linker to a reservoir matrix that serves as a depot for release.

In one aspect, the present invention provides extended release conjugates comprising an insoluble matrix with a multiplicity of covalently attached linker-peptides, wherein the linkers cleave under physiological conditions of pH and temperature to release the free peptide and wherein the peptide is a stabilized GLP-1 agonist which shows degradation of less than 10% over one month at pH 7.4, 37°C. The conjugates of the invention can be illustrated schematically as formula (1)

$$M\text{-}(L\text{-}E)_x \qquad (1)$$

wherein M is an insoluble matrix connected to a multiplicity (x) of GLP-1 agonist peptides E through cleavable linker L. E is a GLP-1 agonist stabilized with respect to degradation that occurs under physiological conditions of pH and temperature to show degradation of less than 10% over one month. x is an integer that represents the number of L-E moieties that yield suitable concentrations in the volume of the matrix. Suitable concentrations are 1-1000 mg peptide per ml matrix. The linker L releases free peptide with a half-life suitable for the desired period of administration.

In a second aspect, the present invention provides linker-peptides L-E having the formula (4)

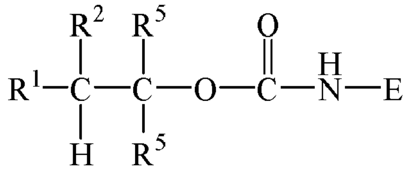

(4)

wherein at least one, or both $R^1$ and $R^2$ is independently CN; $NO_2$;

optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted;
heteroaryl or heteroarylalkyl, each optionally substituted; or $R^3$ is
$OR^9$ or $NR^9_2$ wherein each R is independently H or optionally substituted alkyl, or both $R^9$ groups taken together with the nitrogen to which they are attached form a heterocyclic ring;

$SR^4$ wherein

$R^4$ is optionally substituted alkyl;
aryl or arylalkyl, each optionally substituted; or
heteroaryl or heteroarylalkyl, each optionally substituted;

wherein $R^1$ and $R^2$ may be joined to form a 3-8 membered ring; and
wherein one and only one of $R^1$ and $R^2$ may be H or alkyl, arylalkyl or heteroarylalkyl, each optionally substituted; and
wherein one of $R^5$ is $(CH_2)_yZ$, $(CH_2CH_2O)xCH_2CH_2Z$ or $(CH_2)_yNH$-CO-$(CH_2CH_2O)_xCH_2CH_2Z$, wherein x is 1-100, y = 1-6, and the other $R^5$ is H, alkyl, alkenylalkyl, alkynylalkyl, aryl, arylalkyl, heteroaryl or heteroarylalkyl, each optionally substituted;
Z is a functional group for mediating coupling to the insoluble matrix, and NH is the residue of an amino group of GLP-1 agonist E.

In one embodiment, $R^1$ is CN or $R^3SO_2$, wherein $R^3$ is alkyl or $R^9{}_2N$, wherein each $R^9$ is H, alkyl or substituted alkyl, one $R^5$ is H and the other $R^5$ is $(CH_2)_nZ$ wherein n = 1-6 and Z is a functional group through which the linker-peptide can be attached to M.

In one embodiment, E is [N28Q]exenatide (SEQ ID NO:2). The invention also includes this peptide and any pharmaceutically acceptable salts and pharmaceutical compositions thereof, as well as a protocol for administering a GLP-1 agonist which comprises administering to a subject having a condition benefited by a GLP-1 agonist, a composition that employs this peptide on its salt.

In a third aspect, the invention is directed to protocols for administering the conjugates of formula (1). In one embodiment, the conjugates are prepared as hydrogel microspheres suitable for subcutaneous injection using a narrow-gauge needle. It is expected that the conjugates of the invention are useful for the treatment of metabolic diseases and conditions in both humans and animals. Extended dosages of 1-3 months are achieved.

## Brief Description of the Drawings

Figures 1-6 are schematic representations of various embodiments of the extended-release conjugates of the invention.
Figures 1A and 1B show an overall view of the conjugate in outline form. In Figure 1A one of the components is an 8-arm macromonomer and the other is a 4-arm macromonomer and wherein the linker attached to the GLP-1 agonist is coupled to arms of the 8-arm macromonomer. In Figure 1B, the structure provides for attachment of the linker-agonist to the crosslinkers themselves.
Figure 2 shows more detail of the linkages in Figure 1A.
Figure 3 is a schematic of an embodiment wherein a crosslinking moiety that couples the different macromonomers is provided with a reactive group for attachment of the linker-associated agonist.
Figure 4 shows the matrix of Figure 3 with the linker peptide attached.
Figures 5 and 6 show specific embodiments set forth in Example 4 herein.
Figures 7A-7B show the relationship between the release rate of a drug from a conjugate depot relative to the administration frequency and the relative dose required to achieve a set final concentration ($C_{min}$).
Figures 8A-8C show exenatide and [N28Q]exenatide stability in 200 mM phosphate buffer, pH 7.4, 37°C.
Figure 9 shows the results of peptide isoaspartate methyl transferase (PIMT) assays for iso-aspartate (isoAsp) content in the isolated peaks from the 56 days exenatide degradation reaction shown in Figures 8A and 8B. IsoAsp determinations of exenatide reaction mixture at t = 0 and 56 days, and isolated components of the degradation mix at 56 days. Values for L-Asp- and D-isoAsp-containing peptides were adjusted for the small amounts of L-isoAsp-peptide detected by HPLC in the samples. The residual PIMT-positive peaks at RV 9.9 and 10.4 are attributed to low-level [L-isoAsp$^{28}$]exenatide impurities in the isolated HPLC fractions. Error bars are $\pm$SD.
Figure 10 shows the pharmacokinetics of exenatide in the rat after dosing with a hydrogel-linked unmodified exenatide ($R^1$ = CN; $R^2$ = H). An expected concentration *vs*. time curve was generated (dashed line) based on the results of *in vitro* release kinetics ($t_{1/2}$ = 1400 h) and the known pharmacokinetic parameters of exenatide. Experimental data (squares) are better fit to a model accounting for degradation of exenatide on the hydrogel (solid line) where the overall $t_{1/2}$ = 190 h.
Figure 11 shows the pharmacokinetics of [N28Q]exenatide in the rat after s.c. dosing with hydrogel-linked [N28Q] exenatides. Panel A shows a hydrogel wherein the peptide is linked using L with $R^1$ = $MeSO_2$ and gives $t_{1/2}$ = 350 h. Panel B shows a hydrogel wherein the peptide is linked to L with $R^1$ = CN and gives $t_{1/2}$ = 760 h. Thus plasma levels of [N28Q]exenatide can be maintained for at least one month after a single dose.
Figure 12 shows the comparative results of exenatide and [N28Q]exenatide in an oral glucose tolerance test.

Figure 13 shows the AUC analysis for the oral glucose tolerance test data shown in Figure 7.

Figures 14A-14E show the results of once-monthly dosing of a hydrogel microsphere preparation comprising [N28Q] exenatide ("PL-cmpd") in diabetic ZDF rats.

Figures 15A-15B show the pharmacokinetics of [N28Q]exenatide in rat serum after s.c. dosing of hydrogel microsphere preparations comprising [N28Q]exenatide as described in Example 8A.

Figures 16A-16B show serum [N28Q]exenatide levels after SC injection of mice with microsphere conjugates as described in Example 8B.

## Modes of Carrying Out the Invention

In order to achieve once-monthly administration of a peptide, the peptide must be supplied in the form of an insoluble matrix that is not circulating, but that operates as a depot for release of the drug. Circulating macromolecule conjugates of drugs are unsatisfactory as the conjugates themselves are cleared from the system, e.g., plasma, *per se.* Therefore, the peptide must be supplied within a matrix that is on a macro scale and wherein the peptide (or other drug) is present in the volume of the matrix at a concentration of 1-1000 mg peptide/ml matrix, preferably 1-100 mg peptide/ml matrix and more preferably 1-50 mg peptide/ml matrix. Thus, the matrix is of discernible volume, and may conveniently and collectively be administered in the form of microspheres. (The "volume of the matrix" is the total volume of the dose however supplied, including a dose of microspheres.)

In order to put the nature of the structures involved in the invention into perspective, applicants supply Figures 1-6 that provide an overview of typical embodiments that fall within the scope of the invention.

A depiction of a typical insoluble hydrogel matrix comprising a linked peptide according to the invention is shown in Figure 1A. This is an idealized structure of a matrix formed by crosslinking an 8-arm macromonomer P with a 4-arm macromonomer T with a stoichiometry such that half of the arms of P are crosslinked with arms of T. The remaining non-crosslinked arms of P are connected to linker-peptide. Preparation of hydrogels of this type is described in PCT Publication WO2013/036847, and illustrated as described below in Preparation E.

Figure 1B shows an alternative wherein the releasable linker-drug is coupled to the degradable crosslinker. Figure 1B shows an idealized illustrative structure of a matrix formed by crosslinking a 4-arm macromonomer A, wherein each arm of A is terminated by a group comprising orthogonal first and second functional groups, with a second 4-arm macromonomer B, wherein each arm of B is terminated with a functional group that is reactive with only one of the first or second functional groups of macromonomer A. The remaining functional group of macromonomer A is available for reaction with a linker-peptide comprising a functional group that is reactive with the remaining functional group of the macromonomer. Attachment of the linker-peptide can be performed either prior to gel formation by reaction of macromonomer A with the linker-peptide followed by crosslinking with macromonomer B, or subsequent to gel formation by crosslinking of A and B to form the hydrogel followed by reaction with linker-peptide. As described above, the crosslinking reaction to form the insoluble hydrogel matrix can be performed either as a bulk material or in a suspension or emulsion so as to form a finely-divided particulate polymer, for example microspheres as described in Example 2 herein.

Figure 2 shows a generic structure of a crosslink between an 8-arm macromonomer P and a 4-arm macromonomer T in a matrix further comprising n linker-peptides for each P, as outlined in Figure 1A. An alternative, wherein the linker-agonist is coupled to the crosslinker moieties of the hydrogel is set forth in Figure 1B and Example 4 below.

A generic description of this alternative is provided in Figures 3 and 4. Figure 3 shows an example of derivatization of an insoluble matrix having accessible amine groups with a reagent to introduce cyclooctyne groups. In this example, macromonomer A used in preparation of a matrix as illustrated in Preparation D herein below comprises a lysine residue. Upon formation of the matrix by crosslinking with macromonomer B, the resulting matrix has accessible amine groups suitable for further functionalization, e.g., by reaction with a reagent that introduces a cyclooctyne group.

Figure 4 shows the generic structure of a degradable hydrogel comprising the releasable linker-peptides. Two macromonomers A and B (*e.g.,* as illustrated in Preparation D herein below) are crosslinked, such that each crosslink comprises a releasable linker-peptide. In the diagram, each A and B is coupled by the illustrated crosslink to form an insoluble matrix. Y and Z are connecting functionalities.

Figures 5 and 6 show the structures of the linkages in the extended-release conjugates prepared in Example 4. In Figure 5, the hydrogel matrix comprises crosslinks having degradation controlled by the modulator bis(2-ethoxy)aminosulfonyl, while release of the peptide of SEQ ID NO: 2 from the hydrogel is controlled by the modulator CN. The connecting functionalities are the triazoles resulting from addition of an azide group to the cyclooctyne MFCO.

Figure 6 shows the same structure as Figure 5 except degradation of both the hydrogel crosslinks and release of the peptide from the hydrogel are controlled by the modulator CN and the connecting functionalities are the triazoles resulting from reaction of an azide group to 5-hydroxycyclooctyne.

*Matrix M:*

Matrix M is an insoluble support to which the linker-peptide L-E is attached that serves as the reservoir from which E is released over the duration of treatment. M must be suitable for the attachment of the linker-peptide L-E, or otherwise comprise functional groups that can be derivatized so as to allow for such attachment. M must allow for free diffusion of peptide E once released through cleavage of linker L. M must furthermore be biodegradable to soluble products, and degrade slowly enough to allow for release of E without formation of excessive quantities of soluble M-L-E fragments yet quickly enough to minimize the burden of drug-free M-L remaining after E release in a multiple-dosing scenario.

In one embodiment, M is a biodegradable hydrogel prepared as disclosed in PCT Patent Publication WO2013/036847 and US2014/0288190 both incorporated herein by reference for their description of such hydrogels. These hydrogels comprise beta-eliminative crosslinkers that provide control over the rate of degradation. Thus, in some embodiments, the crosslinkers are of Formula (1) or (2) as follows.

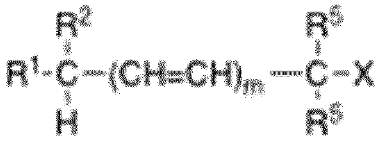
(1)

wherein m is 0 or 1; and
wherein X and one of $R^1$, $R^2$ and $R^5$ each comprise a functional group for coupling to polymer, and
with the proviso that at least one of $R^1$ and $R^2$ is CN; $NO_2$;

- optionally substituted aryl;
- optionally substituted heteroaryl;
- optionally substituted alkenyl;
- optionally substituted alkynyl;
- $COR^3$ or $SOR^3$ or $SO_2R^3$ wherein
  - $R^3$ is H or optionally substituted alkyl;
  - aryl or arylalkyl, each optionally substituted;
  - heteroaryl or heteroarylalkyl, each optionally substituted; or
  - $OR^9$ or $NR^9_2$ wherein each $R^9$ is independently H or optionally substituted alkyl, or both $R^9$ groups taken together with the nitrogen to which they are attached form a heterocyclic ring;
- $SR^4$ wherein
  - $R^4$ is optionally substituted alkyl;
  - aryl or arylalkyl, each optionally substituted; or
  - heteroaryl or heteroarylalkyl, each optionally substituted;

wherein $R^1$ and $R^2$ may be joined to form a 3-8 membered ring; and
wherein any remaining $R^1$ and $R^2$ is H or is alkyl, arylalkyl or heteroarylalkyl, each optionally substituted; and any remaining $R^5$ is independently H or is alkyl, alkenylalkyl, alkynylalkyl, $(OCH_2CH_2)_pO$-alkyl wherein p=1-1000, aryl, arylalkyl, heteroaryl or heteroarylalkyl, each optionally substituted; or
said crosslinker is of formula (2)

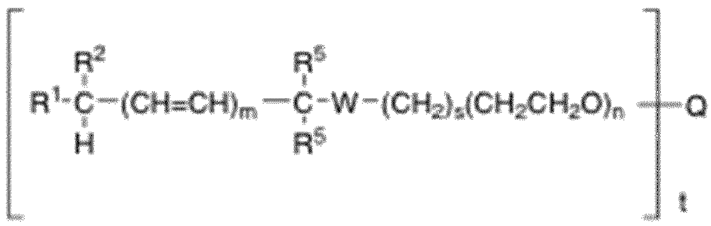
(2)

wherein two of $R^1$, $R^2$ and $R^5$ comprise a functional group for binding to polymer;

m is 0-1;
n is 1-1000;
s is 0-2;
t is 2,4, 8, 16 or 32;
Q is a core group having the valency t;
W is O(C=O)O, O(C=O)NH, O(C=O)S,

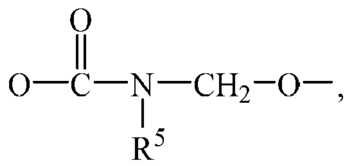

or

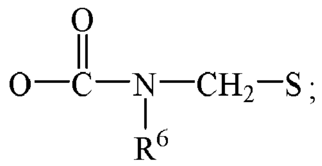

wherein $R^6$ is H, optionally substituted alkyl, optionally substituted aryl, optionally substituted heteroaryl, optionally substituted arylalkyl, or optionally substituted heteroarylalkyl; and
with the proviso that at least one of $R^1$ and $R^2$ is CN; $NO_2$;

- optionally substituted aryl;
- optionally substituted heteroaryl;
- optionally substituted alkenyl;
- optionally substituted alkynyl;
- $COR^3$ or $SOR^3$ or $SO_2R^3$ wherein
  - $R^3$ is H or optionally substituted alkyl;
  - aryl or arylalkyl, each optionally substituted;
  - heteroaryl or heteroarylalkyl, each optionally substituted; or
  - $OR^9$ or $NR^9_2$ wherein each $R^9$ is independently H or optionally substituted alkyl, or both $R^9$ groups taken together with the nitrogen to which they are attached form a heterocyclic ring;
- $SR^4$ wherein
  - $R^4$ is optionally substituted alkyl;
  - aryl or arylalkyl, each optionally substituted; or
  - heteroaryl or heteroarylalkyl, each optionally substituted;

wherein $R^1$ and $R^2$ may be joined to form a 3-8 membered ring; and
wherein any remaining $R^1$ and $R^2$ is H or is alkyl, arylalkyl or heteroarylalkyl, each optionally substituted; and
any remaining $R^5$ is independently H or is alkyl, alkenylalkyl, alkynylalkyl, $(OCH_2CH_2)_pO$-alkyl wherein p=1-1000, aryl, arylalkyl, heteroaryl or heteroarylalkyl, each optionally substituted.

The functional groups used to couple these crosslinkers to the matrix include $N_3$, $NH_2$, NH-$CO_2{}^tBu$, SH, $S^tBu$, maleimide, $CO_2H$, $CO_2{}^tBu$, 1,3-diene, cyclopentadiene, furan, alkyne, cyclooctyne, acrylate, aminooxy, keto and acrylamide. The two functional groups on Formulas (1) and (2) are different from each other, but not cognates. For example, if one is azide, the other is not cyclooctyne or alkyne.

By choosing a beta-eliminative crosslinker that results in M degradation and solubilization at a rate several-fold slower than the rate of E release from cleavage of L-E, the formation of soluble M-L-E fragments is minimized while providing for effective solubilization and clearance of the matrix. In one embodiment of the invention, these hydrogels are prepared by crosslinking multi-arm poly(ethylene glycol)s. The invention further contemplates other useful matrices,

including crosslinked dextrans and hyaluronic acids.

Such matrices may be advantageously made as a slurry of microspheres that is amenable to injection using a narrow-gauge needle. Such slurries may be prepared using known methods, for example either bulk-phase emulsification or more precisely by microfluidic droplet emulsification of prepolymer mixtures. Particle size distribution can be refined through known methods if necessary, for example through sieving.

*Peptide E:*

The peptides (E) that are delivered by the invention are GLP-1 agonists, by which is meant a peptide capable of binding to and activating the GLP-1 receptor. Examples of GLP-1 agonists include the naturally-occurring exendins, for example exenatide (exendin-4; SEQ ID NO:1), liraglutide (SEQ ID NO: 13), lixisenatide (SEQ ID NO:7), taspoglutide (SEQ ID NO:12), and sequence variants thereof. Synthetic sequences that bind and activate the GLP-1 receptor are also contemplated, for example sequences derived by *in vitro* screening and/or selection (Zhang, et al., Nature Commun. (2015) 6:8918).

SEQ ID NO:1 HGEGTFTSDLSKQMEEEAVRLFIEWLKNGGPSSGAPPPS-$NH_2$

[exenatide]

SEQ ID NO:2 HGEGTFTSDL SKQMEEEAVR LFIEWLKQGG PSSGAPPPS-$NH_2$

[N28Q]exenatide

SEQ ID NO:3 HGEGTFTSDL SKQMEEEAVR LFIEWLKAGG PSSGAPPPS-$NH_2$

[N28A]exenatide

SEQ ID NO:4 HGEGTFTSDL SKQMEEEAVR LFIEWLKKGG PSSGAPPPS-$NH_2$

[N28K]exenatide

SEQ ID NO:5 HGEGTFTSDL SKQMEEEAVR LFIEWLKDGG PSSGAPPPS-$NH_2$

[N28D]exenatide

SEQ ID NO:7

HGEGTFTSDLSKQMEEEAVRLFIEWLKNGGPSSGAPPSKKKKKK-$NH_2$

[lixisenatide]

SEQ ID NO:8

HGEGTFTSDLSKQMEEEAVRLFIEWLKQGGPSSGAPPSKKKKKK-

$NH_2$

[N28Q]lixisenatide

SEQ ID NO:9

HGEGTFTSDLSKQMEEEAVRLFIEWLKAGGPSSGAPPSKKKKKK-

$NH_2$

[N28A]lixisenatide

SEQ ID NO:10

HGEGTFTSDLSKQMEEEAVRLFIEWLKKGGPSSGAPPSKKKKKK-

$NH_2$

[N28K]lixisenatide

SEQ ID NO:11 ELVDNAVGGDL SKQMEEEAVR LFIEWLKQGG

PSSGAPPPS-$NH_2$

SEQ ID NO:12 HUEGTFTSDV SSYLEGQAAK EFIAWLVKUR-$NH_2$
[taspoglutide] U = 2-aminoisobutyric acid
SEQ ID NO: 13 HAEGTFTSDVSSYLEGQAAK*EFIAWLVRGRG-OH
K* = Lys(y-Glu-palmitoyl)

It is essential that the peptide agonist E be chemically stable under physiological conditions during the period of administration. While this may not be an issue for direct administration of peptides, given their rapid clearance and frequent administration, extended-release preparations place more stringent requirements on peptide stability. For example, a peptide that degrades under physiological conditions with a half-life of 14 days may be perfectly suitable for once-daily administration as only 5% of the peptide will have degraded in 1 day. The same peptide under extended-release conditions of 30 days (*i.e.*, once-monthly administration) would be 80% degraded by the end of the dosing period, and thus would be unsuitable.

The mechanism of degradation of peptides that contain the sequence Asn-Gly, for example the N28-G29 dipeptide in exenatide is shown below. As shown, an initial L-succinimide is formed which results in conversion of the asparagine residue to aspartic or isoaspartic acid. Both the D and L forms of these modified amino acids are obtained.

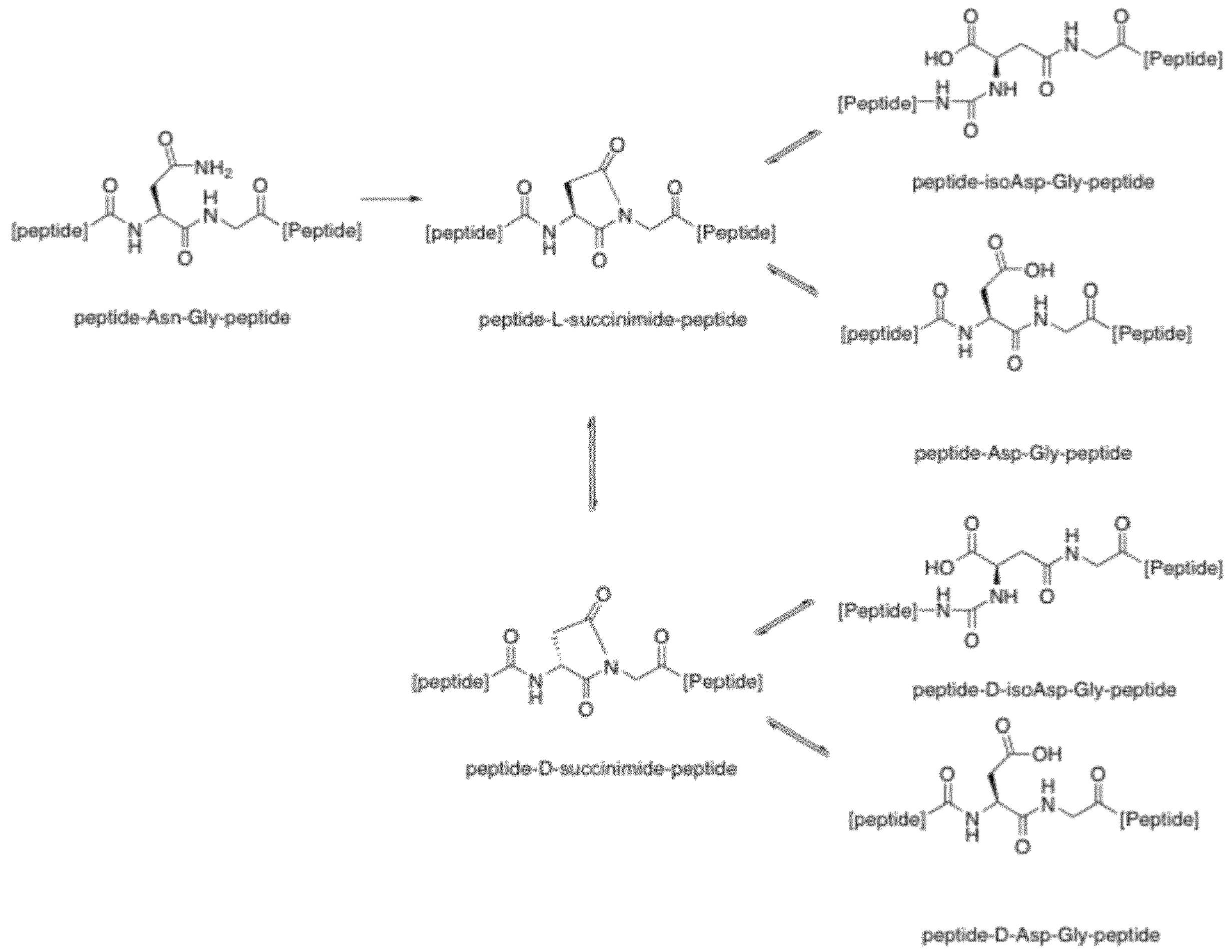

peptide-Asn-Gly-peptide

peptide-L-succinimide-peptide

peptide-isoAsp-Gly-peptide

peptide-Asp-Gly-peptide

peptide-D-succinimide-peptide

peptide-D-isoAsp-Gly-peptide

peptide-D-Asp-Gly-peptide

Examination of sequence variants of exenatide itself has revealed that replacement of N28 with other amino acids can provide an agonist of sufficient stability to support once-monthly administration via an extended-release conjugate while having minimal effects on the potency of the peptide to band and activate the GLP-1 receptor. Thus, agonists having SEQ ID NOS:2-4 wherein N28 of SEQ ID NO: 1 is replaced by Q, A or K, respectively, have been found to bind and activate the GLP-1 receptor with comparable affinity and potency as native exenatide, while showing $<10\%$ or $<9\%$ chemical degradation over a one-month period.

The present invention further contemplates the use of suitably stabilized GLP-1 agonists other than exenatide. The above-described instabilities are expected to occur with other GLP-1 agonists having the Asn-Gly dipeptide sequence, for example lixisenatide and other synthetic peptide sequences. These peptides would be expected to undergo the same sequence of degradation reactions as shown above. Suitably stabilized forms of these GLP-1 agonists useful in the present invention include SEQ ID NOS:8-11. Taspoglutide (SEQ ID NO: 13) and liraglutide (SEQ ID NO: 14) do not have the unstable Asn-Gly dipeptide and are suitable for use in the invention. Other causes of instability may also be corrected. For the agonist of the present invention the stabilized form produces less than 10% degradation products after one month at pH 7.4, 37°C, preferably less than 9% degradation products after one month at pH 7.4, 37°C.

*Cleavable linker L:*

The cleavable linker connects the GLP-1 agonist E to the insoluble matrix M and is cleaved under physiological conditions to release free E. The rate of linker cleavage determines the effective half-life of the peptide and is selected based on the desired frequency of administration. It is further important that the degradation rate of the matrix and the release rate of the peptide be coordinated with the desired frequency of administration. Previously, no attempt has been made to balance these features which balance is necessary for the success of the compositions of the invention in permitting administration on a monthly or less-frequent basis. Any linker which results in achieving this balance will be satisfactory. While there are no hard and fast rules as to the relationship between the rate of release of the drug and the rate of degelation of the matrix, a convenient rule of thumb is that the degelation rate should be approximately three times the rate of release of the free peptide. If the peptide is released too rapidly before degelation occurs, the subject

is left with a deposit of gel at the time of the subsequent dosing. If the release is too slow in comparison to the degelation rate, the peptide remains bound to portions of the gel that are freed into the circulation. Neither circumstance is desirable. This relationship is described by Reid, R., et al. Macromolecules (2015) 48:7359-7369. Structural features that dictate the degelation rate of various matrices depend on crosslinking moieties and structural correlations can be used to provide suitable degelation rate for the matrix.

The balance of degradation and release rates is visualized as follows:

Release of a relatively rapidly-cleared drug from a depot through cleavage of a covalent linker imparts the half-life of the linker cleavage to the half-life of the drug in the plasma. As the dosing frequency is dependent upon drug plasma half-life, there is thus also a relationship between the linker cleavage rate and the dosing frequency. It is typically desired to minimize the difference between the maximum ($C_{max}$) and minimum ($C_{min}$) plasma concentrations of drug to which a patient is exposed in order to reduce the potential for toxicities arising from excessively high drug concentrations while maintaining the amount required for efficacy between doses.

If the dosing frequency is set equal to the plasma half-life of the drug, for example, there will be a 2-fold difference between $C_{max}$ and $C_{min}$, while if the drug is dosed once every 2 plasma half-lives the difference increases to 4-fold. It is thus usual to minimize the number of drug half-lives between doses to minimize $C_{max}$. For an extended-release conjugate, this is achieved by decreasing the release rate.

For an extended-release conjugate, however, the steady-state level of drug from a depot is inversely proportional to the release rate. While the $C_{max}/C_{min}$ ratio may be arbitrarily reduced by slowing the release rate of the drug from the conjugate, the need to maintain a certain value for $C_{min}$ while using an acceptable dosage places a limit on this approach. For a single administration, the dose can be calculated by

$$Dose_{single} = C_{min} \cdot \frac{CL}{F \cdot k_1} \cdot e^{k_1 t_{min}}$$

where CL = drug clearance rate, F = bioavailability, $k_1$ = the rate of drug release from the conjugate depot, and $t_{min}$ = time to reach $C_{min}$. From this it can be shown that the lowest dose required to maintain $C_{min}$ for a given $t_{min}$ is achieved when $k_1 = 1/t_{min}$. Alternatively stated, this is when the drug release half-life (= $\ln(2)/k_1$)=$\ln(2)*t_{min}$. The optimal drug release half-life for a particular dosing frequency is thus given as ln(2)*(dosing interval). When the release half-life is shorter than optimal, the required dose increases due to the depot being depleted prior to reaching $t_{min}$.

Generally speaking, drug release rates that are slower than optimal are more tolerable than ones that are too fast. Thus, a conjugate with a drug release rate supporting once-monthly administration could also be useful for biweekly or once-weekly administration regimens. Further, the relationship between the drug release rate and the required dose to maintain $C_{min}$ is such that some deviation from ideal is tolerable. This is depicted in accordance with the parameters set forth above in Figures 7A and 7B. As shown in Figure 7A, the optimal dose (1) is observed when the ratio of the release half-life to the administration frequency is ln(2) = 0.693. This depiction in Figure 7A, indicates the range of release rates that can be tolerated depending on dosage levels. A higher dosage level will tolerate both a higher release rate since a concentration above the minimum required is still maintained as well as a lower release rate as the drug is provided at a higher level for this longer time. Specifically, if the dosage is increased by up to 10%, release half-lives between 0.45x - 1.1x the dosing frequency are acceptable. If the dosage can be increased by up to 20%, release half-lives between 0.4x - 1.4x the dosing frequency are acceptable. If the dosage can be increased by up to 50%, release half-lives between 0.3x - 2x the dosing frequency are acceptable.

In detail as shown in Figure 7B: for a single dose lasting one month ($t_{min}$ = 720 hours), the optimal drug release rate is 500 hours, yet any release rate between 320 and 800 hours with a ~10% increase in dose, between 280 and 1000 hours with a ~20% increase in dose, or between 220 and 1440 hours with a ~50% increase in dose may also be used. Similarly for a single dose lasting 3 months ($t_{min}$ = 720 hours), the optimal drug release rate is 1500 hours, yet any release rate between 320 and 2400 hours with a ~10% increase in dose, between 840 and 3000 hours with a ~20% increase in dose, or between 660 and 4350 hours with a ~50% increase in dose may also be used. For a single dose lasting 2 weeks ($t_{min}$ = 336 hours), the optimal drug release rate is 230 hours, yet any release rate between 150 and 380 hours with a ~10% increase in dose, between 130 and 470 hours with a ~20% increase in dose, or between 100 and 680 hours with a ~50% increase in dose may also be used.

Similarly, the dose required to maintain the drug concentration above $C_{min}$ at steady-state in a repeat-dosing scenario is given as:

$$Dose_{ss} = C_{min} \cdot \frac{V_{ss} \cdot k_2}{F \cdot k_1} \cdot (e^{k_1 t_{min}} - 1) = C_{min} \cdot \frac{CL}{F \cdot k_1} \cdot (e^{k_1 t_{min}} - 1)$$

In a repeat-dosing scenario, there is no optimal dose as described above, but rather the required dose decreases with decreasing release rate since higher proportions of drug remain from previous doses, thus adding to the total drug depot present.

There are practical limits to the use of slower release rates to decrease dose, however, given a need to minimize the release of drug-bearing gel fragments from the biodegradable matrix, a desire to minimize the total depot burden on the patient, and an increased time to attain steady-state drug levels.

In one embodiment, the cleavable linker L has the formula (3)

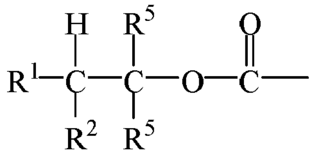
(3)

wherein at least one, or both $R^1$ and $R^2$ is independently CN; $NO_2$;

optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted;
heteroaryl or heteroarylalkyl, each optionally substituted; or $R^3$ is
$OR^9$ or $NR^9_2$ wherein each R is independently H or optionally substituted alkyl, or both $R^9$ groups taken together with the nitrogen to which they are attached form a heterocyclic ring;

$SR^4$ wherein

$R^4$ is optionally substituted alkyl;
aryl or arylalkyl, each optionally substituted; or
heteroaryl or heteroarylalkyl, each optionally substituted;

wherein $R^1$ and $R^2$ may be joined to form a 3-8 membered ring; and
wherein one and only one of $R^1$ and $R^2$ may be H or alkyl, arylalkyl or heteroarylalkyl, each optionally substituted; and wherein one of $R^5$ is $(CH_2)_yZ$, $(CH_2CH_2O)_xCH_2CH_2Z$ or $(CH_2)_yNH\text{-}CO\text{-}(CH_2CH_2O)_xCH_2CH_2Z$, wherein x is 1-100, y = 1-6, and the other $R^5$ is H, alkyl, alkenylalkyl, alkynylalkyl, aryl, arylalkyl, heteroaryl or heteroarylalkyl, each optionally substituted; and
Z is a functional group for mediating coupling to the matrix.

Such linkers cleave by beta-elimination. In preferred embodiments of the invention, $R^1$ is CN or $R^3SO_2$, wherein $R^3$ is substituted or unsubstituted alkyl or $(R^9)_2N$, wherein each $R^9$ is independently substituted or unsubstituted alkyl; $R^2$ is H; one $R^5$ is $(CH_2)_yZ$ and the other $R^5$ is H. Z is $N_3$, SH, $NH\text{-}C(=O)CH_2ONH_2$, or $O\text{-}NH_2$. In particularly preferred embodiments, $R^1$ is CN or $CH_3SO_2$ and/or Z is $N_3$.

Other types of cleavable linkers may be used, for example, linkers that cleave by enzymatic or non-enzymatic hydrolysis, such as those in PCT Publication WO2006/136586 incorporated herein by reference. The sole requirement is that the linker cleavage rate be appropriate for the required administration regimen as described above.

In one embodiment of the present invention, the linker cleaves and releases E with a half-life under physiological conditions suitable to support once-monthly administration, *i.e.*, the linker cleaves and releases E with of half-life between 220 and 1440 hours. In a more preferred embodiment, the linker cleaves and releases E with a half-life between 280 and 1000 hours, more preferably between 320 and 800 hours. In other embodiments of the invention, the linker cleaves

and releases E with a half-life under physiological conditions suitable to support administration once every 3 months or biweekly.

In a specific embodiment of the invention, linker L has the formula (5) wherein $R^1$ is CN. As demonstrated in Example 5, this linker releases peptide from the hydrogel in the rat with a half-life of 760 h.

In another specific embodiment, linker L has the formula (5) wherein $R^1$ is $CH_3SO_2$. As demonstrated in Example 5, this linker releases peptide from the hydrogel in the rat with a half-life of 350 h.

Linker L is connected to peptide E through formation of a carbamate linkage between the C=O group of L and an amino group of E. The amino group may be either the N-terminal alpha-amino group or an epsilon-amino group of a lysine side chain. Methods for preparing both are known in the art. In one embodiment, L is attached to the alpha-amino group of E during solid-phase synthesis of the peptide.

Linker L further comprises a group Z that allows for attachment of the linker-peptide L-E to matrix using chemistry that is compatible and selective in the presence of the functional groups on peptide E. Z may be azide, in which case L-E is connected to the matrix using either a 1,3-dipolar cycloaddition reaction to form a 1,2,3-triazole linkage, or a phosphine-mediated Staudinger ligation to form an amide; both reactions are well-documented in the art. The cycloaddition reaction may be either a copper-catalyzed addition to an alkyne-derivatized matrix or a strain-promoted addition to a cyclooctyne-or bicyclononyne-derivatized matrix. Z may also be an aminooxy or aminooxy-acetamido group, in which case L-E is connected to a keto-derivatized matrix using an oximation reaction. Or Z itself may be a keto group, connecting to an aminooxy group on the matrix. Z may also be a thiol group, in which case L-E is connected to a haloacetyl-derivatized, maleimide-derivatized, or epoxy-derivatized matrix through formation of a thioether.

Thus, the functional groups used to couple L to the matrix include $N_3$, $NH_2$, $NH\text{-}CO_2{}^tBu$, SH, $S^tBu$, maleimide, $CO_2H$, $CO_2{}^tBu$, 1,3-diene, cyclopentadiene, furan, alkyne, cyclooctyne, acrylate, aminooxy, keto or acrylamide.

*Preparation of the conjugates:*

The conjugates are prepared by connecting a peptide E, a cleavable linker L, and a matrix M. In one embodiment, the connections are made pairwise with the order of connection being flexible. Thus, peptide E may be first connected to linker L, and the resulting L-E connected to matrix M. Alternately, linker L may be connected to matrix M, and E then connected to M-L. When M is a matrix prepared by polymerization of monomer units, M-L or M-L-E may be the result of the polymerization process by using a crosslinkable monomer-L or monomer-L-E unit in the reaction.

For biological use, the conjugates must meet stringent criteria for sterility and endotoxin contamination. While in certain cases it may be possible to use a terminal sterilization process, in general the conjugates of the invention are not amenable to this. Insoluble hydrogels, for example, are also not amenable to sterile filtration. Thus, it may be desirable that the conjugates of the invention are prepared under aseptic conditions. The conjugates may be prepared either as injectable microsphere suspensions or they may be formed *in situ* by coinjection of the monomer units.

Formulations:

The conjugates may be formulated using standard pharmaceutically acceptable buffers and excipients to improve injectability and storage stability. Typical formulations include a buffer to maintain pH between 4 and 7, preferably between 5 and 6. Excipients may include stabilizing agents for the peptide drug, for example antibacterial and/or antioxidant agents such as meta-cresol, tonicity-adjusting agents such as a polyol like mannitol, and viscosity-reducing agents such as taurine, theanine, sarcosine, citrulline, and betaine.

Methods of use:

The conjugates of the invention are useful in the treatment of metabolic conditions and diseases in both humans and animals in which the administration of a GLP-1 agonist is known to be effective, including but not limited to type-2 diabetes, metabolic syndrome, and obesity. The greatly increased effective half-life enables once-monthly dosing, thus improving patient compliance (obviating missed doses) and improving patient quality of life. Dosing is preferentially by subcutaneous injection, and may be performed using an autoinjector.

The following examples are offered to illustrate but not to limit the invention.

Preparation A

*In Vitro* Degradation of Exenatide

A solution of 2.4 mM exenatide (1 mL), 0.1% $NaN_3$ and 200 uM Lys(DNP)OH as internal standard in 200 mM $NaP_i$, pH 7.4, was kept at 37°C. At intervals, 50 uL aliquots were removed and frozen at -20°C until assay. Various

samples were thawed and analyzed a) by HPLC, b) for GLP1R agonist activity, and c) for protein isoaspartate methyl transferase activity. The sample incubated for 56 days was subjected to HPLC and samples at RV 9.9, 10.4 and 10.8 were collected and individually analyzed for purity by analytical RP-HPLC, GLP1R agonist and PIMT activities.

**(a)** HPLC profiles of the deamidation of exenatide *vs*. time are shown in Figures 8A-8C. Each peak from the reaction at 56 days was purified by RP-HPLC. Panel A shows HPLC traces obtained at 0 (top), 7, 28, and 56 (bottom) days for native exenatide. The initial single peak for exenatide is gradually replaced by peaks for several degradation products. Panel B shows the time course of the degradation of exenatide as derived from the data in panel A. The decrease in exenatide (squares) and the increase in the major degradation product show a $t_{1/2}$ of approximately 10 days. Panel C shows the HPLC traces obtained with [N28Q]exenatide corresponding to those for exenatide in panel A. Analytical HPLC of isolated degradation products showed that the L-Asp and D-isoAsp peaks contained ~7 and 12% contaminating IsoAsp, respectively; the isolated IsoAsp showed a single peak.

**(b)** Time points were analyzed for GLP-1 receptor activation by GLP1R cAMP Hunter™ bioassay (DiscoverX). The $EC_{50}$ values are summarized below in Table 1.

Table 1

$EC_{50}$ values of exenatide degradation products in GLP1R cAMP Hunter™ assay.

| $Xaa^{28}$ | $EC_{50}$, pM | Xaa/Asn $EC_{50}$ (M) |
|---|---|---|
| Asn, Exenatide (t = 0) | 130 | 1 |
| Product mixture (t = 56 days) | 173 | 1.3 |
| L-Asp (RV 9.8) | 197 | 1.5 |
| D-isoAsp (RV 10.4) | 244 | 1.9 |
| L-IsoAsp (RV 10.8) | 324 | 2.5 |

Peptide concentrations calculated from $A_{280}$ and $\varepsilon_{280}$ = 5,500 $M^{-1}$ $cm^{-1}$; L-Asp and D-isoAsp values uncorrected for the small amounts isoAsp impurity.

The Asp peptide isolated from the deamidation mixture contained a small amount of potentially interfering isoAsp peptide, but synthetic [$Asp^{28}$]exenatide showed agonist activity comparable to exenatide. The amount of D-isoAsp formed in the deamidation reaction at 56 days is so small (~12%) it cannot contribute significantly to agonist activity of the mixture.

**(c)** To assay the presence of isoaspartate, protein isoaspartate methyl transferase (PIMT) assays were performed using the ISOQUANT® isoaspartate detection kit as recommended by the supplier (Promega). Sample mixtures at t = 0 and t = 56 days, as well as individual samples purified from the t= 56 day mixture were assayed for isoAsp peptides. Figure 9 shows the AdoHCys/peptide specific activity formed in a) the total mixture and in b) isolated peaks RV 9.9 and RV 10.4 corrected for small amounts of contaminating isoAsp from the peak with RV 10.8.

Preparation B

Preparation of Exenatide Analogs

The Ala, Asp, Gln and Lys substitutions for $Asn^{28}$ of exenatide were prepared by SPPS. All [$Xaa^{28}$]exenatides had $EC_{50}$ values (17- to 41 pM) comparable to exenatide (17 pM) in a GLP-1RA assay. Upon extended incubation (~3 months) of these [$Xaa^{28}$]exenatides in 200 mM Pi, pH 7.4, 37 °C, major new peaks were not observed, except for [$Asp^{28}$]exenatide which slowly isomerized to [$isoAsp^{28}$]exenatide. At low peptide concentrations (~0.2 mM), small losses were observed in $A_{280}$ consistent with non-specific adsorption to vessel surfaces. At 2 mM [$Gln^{28}$]exenatide, the $t_{1/2}$ for loss of peptide was estimate at >/= 30 weeks. Hence, [$Gln^{28}$]exenatide is very stable under physiological conditions. A $t_{1/2}$ of 30 weeks would give a < 9% loss in one month.

Preparation C

*In Vitro* Receptor Binding and Activity of Exendin Analogs

The ability of various exenatide analogs to activate the GLP-1 receptor was assayed using a cAMP assay (GLP1R cAMP Hunter™ bioassay (DiscoverX)). The N28D, N28A, N28K, and N28Q analogs were found to have comparable ability to exenatide.

| Analog | $EC_{50}$ |
|---|---|
| Exenatide | 29 pM |
| N28D | 41 |
| N28A | 25 |
| N28K | 35 |
| N28Q | 17 |

When the mixture of degradation products (Figure 8A, 56 days) was tested in the cAMP assay, it had an equivalent $EC_{50}$ to the starting exenatide (Figure 8A, 0 days). Likewise, the putative [IsoAsp]$^{28}$-and [Asp]$^{28}$exenatides purified from the mixtures as well as synthetic [Asp]$^{28}$exenatide showed $EC_{50}$ values similar to exenatide. However, when tested in an ELISA assay (Peninsula Lab) the isolated deamidation products and synthetic [N28D]-exenatide showed greatly decreased affinity ($EC_{50}$ exenatide, 0.2 nM; N28A = 6 nM; N28Q = 10 nM; N28D > 100 nM; N28K > 100 nM). Hence, the major degradation products have equivalent agonist activity as exenatide, but would not have been measured by the LC-MS/MS or ELISA assays used for serum exenatide.

Preparation D

Preparation of Degradable Microspheres

*Preparation of Macromonomer A*

**(a)** A solution of 1-(bis-(2-methoxyethyl)aminosulfonyl)-7-azido-2-heptyl succinimidyl carbonate (prepared using the methods described in WO2013/036847; 1.12 mmol) in 5 mL of acetonitrile was added to a solution of H-Lys(Boc)-OH (300 mg, 1.22 mmol) and $NaHCO_3$ (420 mg, 5.0 mmol) in 10 mL of water and 5 mL of acetonitrile. After 0.5 h, the solution was concentrated under vacuum to remove acetonitrile, acidified with 1 N HCl, and extracted with ethyl acetate. The extract was washed with water and brine, then dried over $MgSO_4$, filtered, and evaporated to provide crude Na-[1-(bis-(2-methoxyethyl)aminosulfonyl)-7-azido-2-heptyloxy)carbonyl]-Ne-(BOC)-lysine ("azido-linker[mod] -Lys(Boc)-OH").

Prepared according to this method were azido-linker[mod]-Lys(Boc)-OH wherein the modulator is bis(2-methoxyethyl)aminosulfonyl, dimethylaminosulfonyl, cyano, methylsulfonyl, 4-methylpiperidinylsulfonyl, morpholinosulfonyl or phenylsulfonyl.

**(b)** The above crude azido-linker[mod]-Lys(Boc)-OH was dissolved in 25 mL of $CH_2Cl_2$ and treated with N-hydroxysuccinimide (138 mg, 1.2 mmol) and dicyclohexylcarbodiimide (0.5 mL of a 60 wt% solution in xylenes) for 2 h. The mixture was filtered and chromatographed on $SiO_2$ using a gradient of acetone in hexane to provide the succinimidyl ester azido-linker[mod]-Lys(Boc)-OSu.

Prepared according to this method were azido-linker[mod]-Lys(Boc)-OSu wherein the modulator is bis(2-methoxyethyl)aminosulfonyl, dimethylaminosulfonyl, cyano, methylsulfonyl, 4-methylpiperidinylsulfonyl, morpholinosulfonyl or phenylsulfonyl.

**(c)** A solution of 20-kDa 4-arm PEG-tetraamine in acetonitrile (10 mL, 200 mg/mL, 40 mM amine, 0.4 mmol, 1 equiv) containing N,N-diisopropylethylamine (80 mM, 0.8 mmol, 2 equiv), was treated with a solution of azido-linker[mod] -Lys(Boc)-OSu in acetonitrile (3.3 mL, 145.5 mM, 0.48 mmol, 1.2 equiv). The resulting mixture was kept at room temperature for 1 h, then a 0.010 mL sample was assessed for amine content by TNBS assay using the PEG-tetraamine as a standard; typically <1% of the starting amines remain. The reaction was then treated with acetic anhydride (40.8 mg, 0.0378 mL, 0.4 mmol, 1 equiv) for 15 minutes prior to concentration under vacuum to a viscous syrup (~4 mL) that was slowly added to MTBE (350 mL). The resulting suspension was stirred for 1 h, then the precipitate was recovered by filtration, washed with MTBE (150 mL), and dried under vacuum to give macromonomer A as a white solid.

Prepared according to this method were macromonomer A wherein the modulator is bis(2-methoxyethyl)aminosulfonyl, dimethylaminosulfonyl, cyano, methylsulfonyl, 4-methylpiperidinylsulfonyl, morpholinosulfonyl or phenylsulfonyl.

*Preparation of Macromonomer B*

A 4-mL, screw top vial was charged with $PEG_{20kDa}$-$[NH_2]_4$ (SunBright PTE-200PA; 150 mg, 7.6 μmol PEG, 30.2 μmol $NH_2$, 1.0 equiv, 20 mM final amine concentration), MeCN (1.5 mL), and $iPr_2NEt$ (7 μL, 40 μmol, 1.3 equiv,

27 mM final concentration). A solution of the activated ester cyclooctyne (39 μmol, 1.3 equiv, 27 mM final concentration) was added and the reaction mixture was stirred at ambient temperature. Reactions were monitored by C18 HPLC (20-80%B over 11 min) by ELSD. When complete, $AC_2O$ (3 μL, 30 μmol, 1 equiv per starting $NH_2$) was added to the reaction mixture and the mixture was stirred for 30 min. The reaction mixture was then concentrated to a thick oil and suspended in MTBE (20 mL). The resulting suspension as vigorously stirred for 10 min. The resulting solids were triturated three times with MTBE (20 mL) by vigorously mixing, pelleting in a centrifuge (2800 rpm, 4°C, 10 min), and removal of the supernatant by pipette. The resulting solids were dried under vacuum at ambient temperature for no more than 30 min. Stock solutions were prepared in 20 mM NaOAc (pH 5) with a target amine concentration of 20 mM. Cyclooctyne concentration was then verified by treatment with $PEG_7$-$N_3$ (2 equiv) and back-titration of the unreacted $PEG_7$-$N_3$ with DBCO-$CO_2$H.

Macromonomers prepared using this procedure include those wherein the cyclooctyne group is MFCO, 5-hydroxycyclooctyne, 3-hydroxycyclooctyne, BCN, DIBO, 3-(carboxymethoxy)cyclooctyne, and 3-(2-hydroxyethoxy)cyclooctyne, prepared using MFCO pentafluorophenyl ester, 5-((4-nitrophenoxy-carbonyl)oxy)cyclooctyne, 3-(4-nitrophenoxycarbonyl)oxycyclooctyne, BCN hydroxysuccinimidyl carbonate, DIBO 4-nitrophenyl carbonate, 3-(carboxymethoxy)cyclooctyne succinimidyl ester or 3-(hydroxyethoxy)cyclooctyne 4-nitrophenyl carbonate.

Preparation E

Preparation of Illustrative Hydrogel of Figure 1A

The derivatized 8-arm macromonomer P, is prepared as follows: Macromonomer P is an 8-arm PEG with each arm terminated with a cyclooctyne. A solution of 200 mg of 40-kDa 8-arm PEG-amine•HCl (JenKem Technologies; 40 umol $NH_2$), 20 mg of BCN p-nitrophenyl carbonate (SynAffix; 63 umol), and 20 uL of N,N-diisopropylethylamine (115 umol) in 2 mL of DMF was stirred 16 h at ambient temperature. After quenching with 0.5 mL of 100 mM taurine in 0.1 M KPi, pH 7.5, for 1 h, the mixture was dialyzed sequentially against water, 1:1 methanol/water, and methanol using a 12-kDa membrane. After evaporation, the residue was dissolved in 2 mL of THF and precipitated with 10 mL of methyl tbutyl ether. The product was collected and dried (190 mg). Macromonomers P comprising other cyclooctynes may be prepared similarly by using the appropriate activated cyclooctyne.

The derivatized 4-arm macromonomer T is prepared as follows: T comprises a 4-arm PEG with each arm terminated with a releasable linker-azide. A solution of 25 umol of the azido-linker-succinimidyl carbonate (prepared using the methods described in WO2013/036847) in 1 mL of ACN was added to a mix of 5 umol (100 mg) of 20-kDa 4-arm PEG-amine hydrochloride (pentaerythritol core, JenKem Technologies) in 1 mL of water and 40 uL of 1.0 M $NaHCO_3$ (40 umol). After 1 hr at ambient temperature the solution was dialyzed (12-14 k MWCO) against 1 L of 50% methanol followed by 1 L of methanol. After evaporation, the residue (109 mg) was dissolved in 2.12 mL of sterile-filtered 10 mM NaOAc, pH 5.0, and stored frozen at -20 °C. The azide concentration determined by reaction with DBCO-acid was 9.5 mM. Macromonomers T comprising linker-azides having alternate modulators may be prepared similarly by using the appropriate azide-linker-succinimidyl carbonates.

The peptide-releasing hydrogels may be prepared from the derivatized macromonomers in at least two different ways.

(a) In one embodiment, linker-peptide is attached to macromonomer P prior to formation of the insoluble hydrogel matrix. An azido-linker-peptide of formula (4) such as that illustrated in Example 1 herein is mixed with macromonomer P in a stoichiometry such that some fraction of the arms of P are derivatized with linker-peptide. The resulting material is then crosslinked using sufficient macromonomer T to react the remaining arms of P with arms of T and thus form an insoluble matrix. Thus, n moles of azido-linker-peptide of formula (4) is mixed with n/(8f) moles of macromonomer P, where f = the desired fractional loading of arms with linker-peptide (i.e., for 50% loading of arms, f = 0.5) in a suitable solvent, typically buffered aqueous media. After allowing sufficient time for the azido-linker-peptide to react, the resulting solution is mixed with n(1/f - 1)/4 moles of macromonomer T to form the insoluble hydrogel matrix. Typically, f is chosen such that there are > 3 crosslinked arms to each P residue in the hydrogel matrix (f < 0.625). The crosslinking reaction to form the insoluble hydrogel matrix can be performed either as a bulk material or in a suspension or emulsion so as to form a finely-divided particulate polymer, for example microspheres as described in Example 2 herein.

(b) Alternatively, the insoluble hydrogel matrix can be prepared, followed by attachment of the linker-peptide. For a final hydrogel comprising 8f equivalents of linker-peptide for each P macromonomer residue in the matrix, an insoluble hydrogel matrix is formed by reaction of n/(8f) moles of macromonomer P with n(1/f - 1)/4 moles of macromonomer T. The crosslinking reaction to form the insoluble hydrogel matrix can be performed either as a bulk material or in a suspension or emulsion so as to form a finely-divided particulate polymer, for example microspheres as described in Example 2 herein. The polymerized matrix is then allowed to react with a solution of at least n moles of azido-linker-peptide of formula (4), such that the linker-peptide is covalently attached to the matrix. Unreacted azido-linker-peptide is washed from the matrix to provide the peptide-releasing hydrogel.

Example 1

Preparation of Azido-linker-[N28Q]exenatides of Formula (4) wherein $R^1$ = CN or $MeSO_2$; $R^2$ = H; one $R^5$ = H and the other $R^5$ = $(CH_2)_5N_3$; P = $N^{\alpha}$-[N28Q]exenatide

Peptides were synthesized by standard solid-phase methodology using Chemmatrix® Rink amide resin (0.5 meq/g) on a Symphony® peptide synthesizer. Fmoc-amino acids (5 eq per coupling) were double-coupled to the N-terminus of the peptide chain using HCTU (4.9 eq per coupling) and N,N-diisopropylethylamine (10 eq per coupling) in DMF at ambient temperature. Fmoc groups were removed using 20% 4-methylpiperidine in DMF. [N28Q]exenatide was deprotected and cleaved from the resin using 95:2.5:2.5 trifluoroacetic acid/triisopropylsilane/dithiothreitol.

Crude [N28Q]exenatide (22 mg) was purified on a semi-preparative scale using a Shimadzu™ LC-20AD system equipped with a Peak Scientific HiQ® 5 μ C18 column (50 x 20 mm ID) eluting with a linear gradient of 30%-60% MeCN (0.1% TFA) in water (0.1% TFA). The purest fractions, as judged by analytical C18 HPLC, were combined, concentrated by ~40% to remove MeCN, and lyophilized to provide [N28Q]exenatide (6.4 mg, 1.4 mmol) as a fluffy white solid. C18 HPLC purity determined at 280 nm: 86% pure (RV = 9.4 min); [N28Q]exenatide; m/z = 4200.

On-resin N-terminal carbamoylation of peptides was performed by a modification of a previously described method (Schneider, E. L. et al, Biocong. Chem (2016) 1 March on line prepublication) and is exemplified by the following.

*$N^{\alpha}$-(7-Azido-1-cyano-2-heptyloxycarbonyl)-[$Gln^{28}$]exenatide.* In a septum-capped 125 mL Erlenmeyer flask, $NH_2$[N28Q]exenatide (free α-amine) Chemmatrix® Rink amide resin (0.5 meq/g substitution, 0.48 mmol peptide/g peptide-resin, 4.00 g peptide-resin, 0.48 mmol peptide) was gently stirred in 40 mL of DMF for 30 min at ambient temperature under $N_2$. The swollen resin was then treated with 8 mL of *O*-(7-azido-1-cyano-2-heptyl)-*O'*-succinimidyl carbonate (0.18 M in DMF, 1.44 mmol, 30 mM final) and 4-methyl-morpholine (158 μL, 1.44 mmol). The reaction mixture was gently stirred under $N_2$ for 2 h then vacuum filtered. The resin was washed with successively DMF (3 x 30 mL) and $CH_2Cl_2$ (4 x 50 mL) then dried under high vacuum. Kaiser test was negative for free amines in intermediate linker-modified resin (3.84 g). The resin was then treated with 40 mL of 90:5:5 TFA:TIPS:$H_2O$ with stirring under $N_2$. After 2.5 h, the resin was vacuum filtered and washed with TFA (2 x 10 mL). The filtrate was concentrated to ~20 mL. The crude linker-peptide was precipitated by drop-wise addition of the TFA concentrate to ice-cold $Et_2O$:hexane (2:1, 160 mL) in 4 tared 50 mL Falcon tubes. After incubating on ice for 30 min, the crude linker-peptide was pelleted by centrifugation (3 min at 2000 x g), and the supernatant was decanted. The pellet was triturated/vortexed with ice-cold $Et_2O$:hexane (2:1, 160 mL), incubated on ice, centrifuged, and decanted as above. After drying under high vacuum, the crude linker-peptide was isolated as an off-white solid (1.76 g) then dissolved in 5% AcOH. After heating at 40°C for 1 h, the crude material was purified by preparative HPLC. MS: m/z = 4408.

$N^{\alpha}$-[1-(methylsulfonyl)-7-azido-2-heptyloxycarbonyl]-exenatide was similarly prepared using *O*-(7-azido-1-(methylsulfonyl)-2-heptyl)-*O'*-succinimidyl carbonate. MS: m/z = 4461.

Example 2

Preparation of PEG Hydrogel Microspheres

A 2-reagent Telos® hydrophobic flow-focusing microfluidic chip (Dolomite) with seven parallel 50 um drop forming channels was used. Fluid flow was controlled by a gas-pressure driven pump, similar in function to the Mitos Pressure Pumps manufactured by Dolomite Microfluidics. These pumps use pressurized gas to drive the flow of liquid through the microfluidic chip. The driving pressure is computer controlled using proportional pressure regulators (Proportion Air, MPV series) to maintain a stable flow rate by using a feedback loop from a liquid flow sensor (Sensirion, SLI-0430). This type of flow control is scalable to deliver liquid from multi-liter reservoirs, and produces flow rates with ~1% standard deviation, superior to syringe pumps that often have up to a 20% oscillation in their flow rate. This system was used to deliver the two hydrogel prepolymer solutions and the continuous phase. Typical flow rates were 2.1 ml/h for each prepolymer solution and 14 mL/h for the continuous phase. The continuous phase was composed of decane containing 1% w/v Abil® EM90 (Evonik) and 1% w/v PGPR (Danisco). The outlet tube of the device was connected to a fraction collector (Gilson FC203B), and fractions were collected in 10 minute intervals. Quality control was performed by photographing the chip at 5x magnification with a high speed camera (UniBrain®, Fire-I 580b) attached to a microscope (Nikon™, EQ-51436) equipped with an automated stage to visualize the seven channels of the chip. Images of each channel were collected every 5 minutes. Fractions containing large particles resulting from device failure could be eliminated from the batch.

Microspheres washes were conducted in 50 mL Teflon® (FEP) centrifuge tubes (Oak Ridge, 3114-0050). After

washing, the microspheres were recovered by centrifugation. Centrifugation is conducted for 5 min at 3000 g's for separation of organic phases, and 20 min for separation of aqueous phases. All solutions and wash solvents were filtered through 0.2 um Nylon-66 filters (Tisch, SPEC17984).

A suspension of microspheres from a microfluidics run (30 mL) in decane containing surfactant were allowed to cure at room temperature for 24 h. The decane layer was removed, and the microspheres were partitioned between 0.1% (w/v) aqueous $NaN_3$ (15 mL) and pentane. The mixture was agitated for 30 min then the pentane phase was separated by centrifugation. The microsphere suspension was then treated with water (30 mL) and washed with five consecutive (39 mL) portions of pentane. After centrifugation, the excess aqueous phase was removed and the microsphere slurry was treated with an equal volume of 50 % w/v TFA for 30 min for sterilization. The microspheres were recovered by centrifugation at 1000 g's (note: the spheres shrink in TFA and form a compact pellet, so excessive force should be avoided). The pellet was treated with 0.125 M $Na_2HPO_4$ (150 mL) to give a suspension of pH ~6.5. After swelling for 18 h the spheres were recovered by centrifugation, then washed with five 100 mL portions of water and finally five 100 mL portions of 70% ethanol. The slurry was pelleted to final concentration at 3000 g's for 30 min. After aspiration of the supernatant, the microsphere slurry was transferred to a 60 mL syringe (BD No. 309653) that was connected by a Luer coupling to another 60 mL syringe and homogenized by several back-and-forth passages to disperse small clumps. The syringe containing the slurry was used to load individual 10 mL syringes through the Luer coupling that were stored at 4°C until use.

Three 0.100 mL portions of amino-microsphere slurry in acetonitrile were weighed to determine their density (0.79 ± 0.2 g/mL), then each portion was then treated with 0.900 mL of 50 mM NaOH for 18 h at room temperature to cleave crosslinks and form $[H_2N\text{-Lys}(NH_2)\text{-NH}]_4\text{-PEG}_{20kDa}$ monomers. Each sample was assayed for total amine concentration by TNBS assay by diluting 0.030 mL to 0.120 mL with borate buffer (100 mM, pH 9.3) then treating with 0.150 mL of borate buffer containing 0.04% w/v sodium 2,4,6-trinitrobenzenesulfonate in a microtiter plate. The change in absorbance of the TNBS reactions at 420 nm was monitored for 3 h in a plate reader at 25°C then the final absorbance at 420 nM was recorded. Equivalent reactions containing TNBS alone were used for background subtraction and reactions containing 40, 20, or 10 uM lysine were used for amine concentration standards. The total amine concentration/2 of the microsphere digests provides the free e-amine content of the gel.

## Example 3

### Preparation of Cyclooctyne-Microspheres

The reaction is performed in the syringe reaction vessel as follows. For each 4 mL of a packed suspension of amino-microspheres in MeCN containing 2 μmol amine/mL gel slurry are added 32 μmol DIPEA (4 equivalents) in 1 mL MeCN, and 9.6 μmol (1.2 equivalents) of 1-fluoro-2-cyclooctyne-1-carboxylate pentafluorophenyl ester (MFCO-PFP) in 1 mL MeCN. After 1 h rocking at ambient temperature, a small amount (~50 uL) of microspheres is expelled from the syringe outlet and treated with 0.5 mL of 0.04% w/v TNBS in 0.1 M sodium borate, pH ~9.3 (1) for 30 min; complete reaction is indicated by a microsphere color matching the TNBS solution compared to starting amino-microspheres which stain an intense orange. After reaction, the microspheres are capped by the addition of 8 μmol (1 equivalent) of $AC_2O$ in 1 mL MeCN for 10 min. After removal of the supernatant, ~2 mL of the microspheres is transferred to a second 10 mL syringe, each slurry is washed with 4 x 3 volumes MeCN per packed slurry volume and the slurries combined.

## Example 4

### Preparation of Peptide-Releasing PEG Hydrogel Microspheres

The coupling of the azido-linker-[N28Q]exenatides prepared in Example 1 was performed in the syringe reaction vessel described in (Schneider, *et al, supra*). To a suspension of 2.4 g of a slurry of MFCO-derivatized microsphere of Example 3 (11.2 μmol MFCO) in 30% MeCN in a 10 mL syringe was added a solution of 46 mg (10.4 μmol) of $N^{\alpha}$-[1-(methylsulfonyl)-7-azido-2-heptyloxycarbonyl]-[N28Q]exenatide in 2 mL 30% MeCN. The mixture was slowly rotated until the $OD_{280}$ of an aliquot was constant at ~24 hr. About 50% of the slurry was transferred to a second syringe, and both samples were washed with 4 x 2 mL of 30% MeCN and then 5 x 5 mL of 10 mM $NaP_i$, 0.04% Tween® 20, pH 6.2. The [N28Q]exenatide-loaded microspheres were then syringe-to-syringe transferred to several 1.0 mL dosing syringes. The total loading of the microsphere was 1.9 μmol peptide $gm^{-1}$ of slurry as determined by the total peptide released at pH 8.4.

Release of free [N28Q]exenatide from the microspheres was measured *in vitro* by suspending a sample of the conjugate in 0.1 M borate, pH 9.4, and following the solubilization by the increase in $OD_{280}$ at 37°C (Figure 5). A first-order release was observed showing a half-life = 6.7 h at pH 9.4. This extrapolates to 670 h at pH 7.4. Microspheres loaded with $N^{\alpha}$-[1-cyano-7-azido-2-heptyloxycarbonyl]-[N28Q]exenatide were prepared similarly. Release of free [N28Q] exenatide from these microspheres was measured *in vitro* by suspending a sample of the conjugate in 0.1 M borate,

pH 9.4, and following the solubilization by the increase in $OD_{280}$ at 37°C. A first-order release was observed showing a half-life = 16.5 h at pH 9.4. This extrapolates to 1650 h at pH 7.4.

Microspheres comprising [N28Q]exenatide attached via a linker wherein $R^1$ = CN and $R^2$ = H were prepared similarly, using Nα-[1-cyano-7-azido-2-heptyloxycarbonyl]-[N28Q]exenatide (Example 1). The final preparation comprised 2.2 μmol peptide $gm^{-1}$ in isotonic acetate buffer (10 mM acetate, 120 mM NaCl, pH 5.0) with 0.05% Tween® 20.

Example 5

Rat Pharmacokinetics

The contents of tared 1 mL dosing syringes containing the microsphere slurries prepared in Example 4 were administered through a 27 gauge needle s.c. into the flank of cannulated male Sprague Dawley rats, ~350 g. Each syringe contained 0.45 or 0.98 μmol peptide at 1.9 μmol peptide $g^{-1}$ slurry. The syringes were weighed prior to and after dosing to verify the mass delivered to each rat. Blood samples (300 μL) were drawn and the serum was frozen at -80°C until analysis.

Exenatide concentrations were measured by ELISA according to the manufacturer's protocol (Peninsula Laboratories Inc., #S-1311). Frozen serum samples were thawed on ice and diluted between 5 and 20-fold in the provided rat serum. The standard exenatide showed an $EC_{50}$ = 0.22 nM (reported 0.19 nM). Data replicates were averaged and fitted to appropriate pharmacokinetic models. [N28Q]Exenatide concentrations in sera were measured by LC-MS/MS.

The results are shown in Figures 10 and 11.

Figure 10 shows the pharmacokinetics of exenatide in the rat after dosing with a hydrogel-linked (same as those in Example 4 except using exenatide that is in the native form and where $R^1$ = $MeSO_2$). An expected concentration *vs*. time curve was generated (dashed line) based on the results of *in vitro* release kinetics ($t_{1/2}$ = 1400 h) and the known pharmacokinetic parameters of exenatide. Experimental data (squares) show a better fit to a model accounting for degradation of exenatide on the hydrogel (solid line) where the overall $t_{1/2}$ = 190 h.

Figure 11 shows the pharmacokinetics of [N28Q]exenatides of Example 4 in the rat after s.c. dosing. Panel A shows a hydrogel wherein the peptide is linked using L wherein $R^1$ = $MeSO_2$ and gives $t_{1/2}$ = 350 h. Panel B shows a hydrogel wherein the peptide is linked using L wherein $R^1$ = CN and gives $t_{1/2}$ = 760 h. Thus plasma levels of [N28Q] exenatide can be maintained for at least one month after a single dose, while the data in Figure 11 show a much shorter $t_{1/2}$.

Example 6

Oral Glucose Tolerance Test

The ability of [N28Q]exenatide to provide tolerance to a bolus of oral glucose relative to exenatide was determined in mice. A total of 54 male, 8-week old C57BL/6J mice (JanVier France) were acclimatized for 2 weeks then stratified into 9 groups (n = 6). On day 0, mice were fasted for 4 h then dosed with test article by subcutaneous injection at t = -15 minutes followed by glucose at 0 minutes. Blood glucose was measured at -60, -15, 0, 15, 30, 60, and 120 minutes. Blood samples for insulin measurements were taken at 0 and 15 minutes. Results are shown in Figures 12 and 13. Exenatide and [N28Q]exenatide showed comparable activity in the oral glucose tolerance test.

Figure 12 shows the comparative results of exenatide and [N28Q]exenatide in an oral glucose tolerance test. A clear and similar dose response was observed for exenatide and [N28Q]exenatide in an oral glucose tolerance test in C57BL/J mice dosed at -30 min with exendin-4 and [N28Q]exenatide in 5 different concentrations. Each line represents a significant difference from vehicle. Two-way repeated measurement ANOVA compared to vehicle. $P<0.05$ Bonferroni post hoc test. Glucose bolus was 2 g/kg in 10 ml, per oral.

Figure 13 shows the AUC analysis for the oral glucose tolerance test data shown in Figure 7. A clear and similar dose response was observed for exenatide and [N28Q]exenatide in area under the curve data after an oral glucose tolerance test (OGTT). One-way ANOVA, Bonferroni post hoc test *vs*. vehicle. ***$p < 0.001$.

Example 7

Chronic Glucoregulatory Effects of Hydrogel Microsphere-Conjugated [N28Q]Exenatide Injected Q4Wk *vs*. Exenatide Continuous Infusion

A total of 55 male Zucker diabetic fatty rats (ZDF-$Lepr^{fa}$/Crl) 6 weeks of age, and 180-200 gram (Charles River, USA) were single-housed and blood glucose and body weight were monitored bi-weekly for 2-4 weeks. Based on morning-fed blood glucose, outliers were excluded and 40 diabetic rats (average weight 340 g, blood glucose 9.7 to 22.5 mM, average 16.2 mM) were stratified into 4 groups of n=10.

At t = 0:

Group I received vehicle in an Alzet® pump (2ML4);
Group II received exenatide Alzet® pump (30 μg/kg/day, in AcOH, pH 4.5);
Group III received s.c. hydrogel microsphere-[N28Q]exenatide having drug-release modulator $R^1$ = CN described in Example 4, 0.37 mg peptide) plus vehicle pump;
Group IV received s.c. hydrogel microsphere-[N28Q]exenatide (3.7 mg peptide) plus vehicle pump.

On day 29, two days after the 4 week OGTT, pumps were replaced and the hydrogel microsphere-[N28Q] exenatide was re-dosed s.c. at the same levels. On day 56, the pumps were removed and animals were allowed to recover for 4 wks. Six rats that became diabetic at a later age received [N28Q]exenatide in osmotic pumps (30 μg/kg/day, in AcOH, pH 4.5) on day 28 that were discontinued on day 56, after which animals were allowed to recover for 4 weeks.

Body-weights were monitored daily from day -3 throughout the study. Food and water intake were monitored on day -3 and daily for the first 11 days after the first dose then bi-weekly for the rest of the study period. Blood sampling was performed for pharmacokinetic studies 2 days after the first dose and then once weekly. HbAlc was measured day -3, and days 26 and 55 before OGTT, and the gastric emptying test was performed on day 26. For the OGTT, rats were semi-fasted overnight (60%) then PO glucose (2 g/kg in 10 mL) was administered at t = 0. Blood glucose and insulin were measured at t = -60, -15, 0, 15, 30, 60 and 120 minutes after the glucose challenge. Gastric emptying was measured by administration of acetaminophen (100 mg/kg) with the OGTT on day 26, and blood levels were measured at 15, 30, 60 and 120 min.

The results are shown in Figures 14A-14E. In these figures, for vehicle control (black), exendin-4 delivered by continuous infusion using a subcutaneous pump at 30 ug/kg/day (pink), PL-cmpd dosed at 220 nmol peptide/kg (gray), PL-cmpd dosed at 2200 nmol peptide/kg (blue), and [N28Q]exenatide free peptide delivered by continuous infusion using a subcutaneous pump at 30 ug/kg/day (green).

Figure 14A shows body weights.

Figure 14B shows blood glucose in mmol/L.

Figure 14C shows the results of oral glucose tolerance tests performed at 4 and 8 weeks.

Figure 14D shows levels of glycosylated hemoglobin HbAlc at 4 and 8 weeks.

Figure 14E shows levels of peptides in plasma as a function of time.

A single dose of a hydrogel microsphere preparation comprising [N28Q]exenatide was effective at controlling blood glucose for at least one month.

Example 8

Pharmacokinetics of Hydrogel-Microsphere Conjugates in the Rat and Mouse

**A.** Syringes (0.5 mL U-100 insulin syringe with fixed 29 g x ½" needle, BD) were filled under sterile conditions with the [N28Q]exenatide-microsphere slurry prepared in Example 4 in isotonic acetate (10 mM Na Acetate, 143 mM NaCl) pH 5.0 0.05% Tween® 20. Microspheres using drug-release modulator $R^1$ = $MeSO_2$ contained 1.3 μmol peptide/g slurry, and microspheres using drug-release modulator $R^1$ = CN contained 1.4 μmol peptide/g slurry. The content of each syringe was administered SC in the flank of six cannulated male Sprague Dawley rats (average weight 270 g).

The needle assembly was purged of air and weighed prior to and following dosing to determine the mass of the slurry delivered to each rat; with the $MeSO_2$ modulator 130 mg slurry containing 0.7 mg peptide (170 nmol) was administered to each rat, and with the CN modulator 400 mg slurry containing 2.5 mg peptide (580 nmol) was administered.

Blood samples (300 μL) were drawn at 0, 1, 2, 4, 8, 24, 48, 72, 120, 168, 240, 336, 432, 504, 600, 672 hours for both linkers and additional samples were obtained at 840, 1008, 1176, 1344, 1512, 1680, 1848, and 2016 hours for the linker with the CN modulator. Serum was prepared and frozen at -80°C until analysis. Serum [N28Q]exenatide was analyzed by LC/MS/MS.

The results are shown in Figures 15A and 15B. Figure 15A shows the results after injection with hydrogel-[N28Q] exenatide microspheres with a drug release modulator $R^1$ = $MeSO_2$ (170 nmol [N28Q]exenatide/rat or 2.6 mg/kg); $t_{1/2,\beta}$ was 310 hr. Figure 15B shows the results after injection with hydrogel-[N28Q]exenatide microspheres with a drug release modulator $R^1$ = CN (600 nmol [N28Q]exenatide/rat or 8.9 mg/kg); $t_{1/2,\beta}$ was 880 hr. Error bars are ±SEM.

**B.** The small amounts of microspheres needed in the mouse required a diluent to allow accurate dosing. Using a dual syringe-based reaction vessel {Schneider, 2016 #24} the buffer of a [N28Q]exenatide-microsphere slurry (~1 mL for drug-release modulator $R^1$ = $MeSO_2$, 4 mL for $R^1$ = CN) was aseptically exchanged for a solution of isotonic acetate pH 5.0 (10 mM NaOAc, 143 mM NaCl), 25% glycerol and 0.05% Tween® 20. This diluent served to keep the microspheres in a homogeneous suspension prior to and during administration and allowed dosing of convenient volumes. The slurry was then diluted with the same mixture to give 264 nmol peptide/mg slurry ($R^1$ = $MeSO_2$) or 720 nmol peptide/mg slurry

[$R^1$ = CN). Syringes (0.5 mL U-100 insulin syringe with fixed 29 g x ½" needle, BD) were filled with the suspended microspheres under aseptic conditions. The needle assembly of each syringe was purged of air and weighed prior to and following dosing to determine the average mass of slurry delivered to each mouse.

For $R^1$ = $MeSO_2$, 120 mg of slurry containing 130 ug peptide (30 nmol) was administered SC in the flank of each of 18 CD-1 mice (average weight 30 g). Blood samples (100 μL) were drawn from the orbital sinus at 8, 24, 48, 72, 96, 120, 168, 240, 336, 408, 504, 576 and 672 hours on a staggered schedule to give 6 replicates at each time-point, and sera of each were prepared. For $R^1$ = CN, 200 mg slurry containing 605 μg peptide (144 nmol) was likewise administered SC to 24 CD-1 mice. Blood samples (100 μL) were drawn from the orbital sinus at the same times as above, and also at 840, 1008, 1176, 1344, 1512, 1680, 1848, and 2016 hours on a staggered schedule to give 6 replicates at each time-point. Serum was prepared and frozen at -80°C until analysis. Serum [N28Q]exenatide was analyzed by LC/MS/MS.

Serum samples were treated with 3 vol of ACN and centrifuged. The supernatant was dried, reconstituted and applied to a HPLC MS/MS system. The sample was eluted by a water/ACN gradient containing 0.1% formic acid. The calibration curve for [N28Q]exenatide was linear over the range of 0.25-100 ng/mL. HPLC-MS/MS analyses were carried out on a Sciex 5500 QTrap® mass spectrometer coupled with a Shimadzu HPLC system. The Shimadzu HPLC system consisted of two LC-30AD HPLC pumps and a SIL-30AC autosampler with a 100-μL loop installed. The chromatographic separations were achieved on a 3-μm C18, 2.1 × 50 mm HPLC column, with mobile phase gradients. The mass spectrometer was operated in positive electrospray ionization mode and the resolution setting used was the unit for both Q1 and Q3. The multiple-reactions monitoring (MRM) transition was m/z = 841.1 ->396.3 for [N28Q]exenatide. Peak-area integrations were performed using Analyst software (version 1.5.2) from Sciex.

The results are shown in Figures 16A and 16B. Figure 16A shows the results after injection with hydrogel-[N28Q] exenatide microspheres with a drug release modulator $R^1$ = $MeSO_2$ (30 nmol [N28Q]exenatide/mouse or 4.2 mg/kg). Figure 16B shows the results after injection with hydrogel-[N28Q]exenatide microspheres with a drug release modulator $R^1$ = CN (144 nmol [N28Q]exenatide/mouse or 20.2 mg/kg). Early points were insufficient to calculate absorption phase kinetics, and were not used in fitting the β-phase shown. Error bars are ±SEM.

C *vs.* t curves (text) were analyzed using GraphPad Prism by non-linear regression of eq X (text) with weighting by 1/SD2; since ka >>k1 we modeled the single exponential phase of the terminal half-life using data points later than the first few days.

Table 2

Pharmacokinetic Properties of Hydrogel-[N28Q]Exenatide Microspheres in the Mouse and Rat

| | Mouse | | Rat | |
|---|---|---|---|---|
| Modulator | $MeSO_2$- | -CN | $MeSO_2$- | -CN |
| Dose, μmol/kg | 0.94 | 4.5 | 0.69 | 2.1 |
| k x $10^4$± SE, $h^{-1}$ | 28.4 ± 3.9 | 9.5 ± 1.0 | 22.3 ± 1.6 | 7.8 ± 1.3 |
| $t_{1/2}$, hr | 244 | 730 | 310 | 883 |
| $C_{max,0}$, nM | 2.2 | 0.81 | 1.5 | 0.36 |
| $AUC_{inf}$, nM-hr | 841 ± 275 | 800 ± 323 | 786 ± 37 | 448 ± 91 |
| *Dose adjusted:* | | | | |
| $C_{max,0,}$ nM/(μmol/kg) | 2.3 | 0.18 | 2.2 | 0.17 |

Embodiments of the invention

1. An extended-release conjugate of a GLP-1 agonist comprising an insoluble matrix with a multiplicity of covalently attached linker-agonist peptide, wherein the linkers cleave under physiological conditions of pH and temperature to release free peptide agonist and wherein the agonist peptide of said linker-agonist peptide is said GLP-1 agonist that shows a degradation of less than 10% over one month under physiological conditions of pH and temperature.

2. The extended-release conjugate of embodiment 1 having the formula

$$M\text{-}(L\text{-}E)_x$$

wherein M is an insoluble matrix; L is a cleavable linker having a half-life of cleavage at 37°C, pH 7.4, of between 320 and 2400 hours; and E is a GLP-1 agonist that shows less than 10% chemical degradation after 1 month at pH 7.4, 37°C and x is an integer representing the number of L-E units required to provide a concentration of 1-1000 mg peptide in one ml of the volume occupied by the matrix.

3. The extended-release conjugate of embodiment 1 wherein the GLP-1 agonist is a stabilized exendin comprising an amino acid substitution at the position corresponding to N28 in a native exendin sequence, wherein the native exendin consists of SEQ ID NO:1 or SEQ ID NO:7.

4. The extended-release conjugate of embodiment 3 wherein the GLP-1 agonist is N29D, N28A, N28K or N28Q substituted SEQ ID NO: 1 or SEQ ID NO:7.

5. The extended-release conjugate of embodiment 4 wherein the GLP-1 agonist is SEQ ID NO:2 or SEQ ID NO:8.

6. The extended-release conjugate of any of embodiments 1-5 wherein the insoluble matrix is a biodegradable crosslinked hydrogel comprising polymers coupled by crosslinkers.

7. The extended-release conjugate of embodiment 6 wherein the hydrogel is a biodegradable crosslinked poly(ethylene glycol).

8. The extended-release conjugate of embodiment 6 wherein the hydrogel is in the form of microspheres.

9. The extended-release conjugate of embodiment 6 wherein the polymers comprising the hydrogel are crosslinked by crosslinkers that are cleaved by beta elimination.

10. The extended-release conjugate of embodiment 9 wherein said crosslinkers contained in the hydrogel are of formula (1)

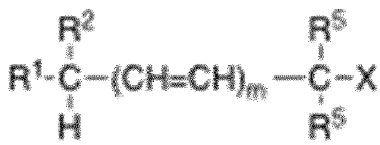

(1)

wherein m is 0 or 1; and
wherein X and one of $R^1$, $R^2$ and $R^5$ is coupled to a polymer contained in the hydrogel,
with the proviso that at least one of $R^1$ and $R^2$ is CN; $NO_2$;

optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted;
heteroaryl or heteroarylalkyl, each optionally substituted; or
$OR^9$ or $NR^9_2$ wherein each $R^9$ is independently H or optionally substituted alkyl, or both $R^9$ groups taken together with the nitrogen to which they are attached form a heterocyclic ring;

$SR^4$ wherein

$R^4$ is optionally substituted alkyl;
aryl or arylalkyl, each optionally substituted; or
heteroaryl or heteroarylalkyl, each optionally substituted;

wherein $R^1$ and $R^2$ may be joined to form a 3-8 membered ring; and
wherein any remaining $R^1$ and $R^2$ is H or is alkyl, arylalkyl or heteroarylalkyl, each optionally substituted; and

any remaining $R^5$ is independently H or is alkyl, alkenylalkyl, alkynylalkyl, $(OCH_2CH_2)_p$ O-alkyl wherein p=1-1000, aryl, arylalkyl, heteroaryl or heteroarylalkyl, each optionally substituted; or
said crosslinkers when contained in the hydrogel are of formula (2)

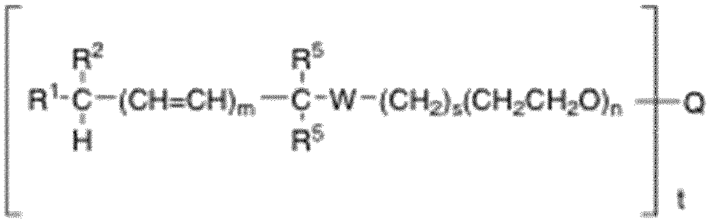

(2)

wherein two of $R^1$, $R^2$ and $R^5$ are coupled to a polymer contained in the hydrogel;
m is 0-1;
n is 1-1000;
s is 0-2;
t is 2,4, 8, 16 or 32;
Q is a core group having the valency t;
W is O(C=O)O, O(C=O)NH, O(C=O)S,

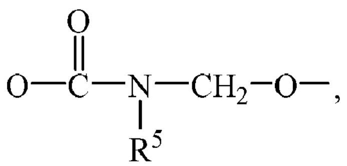

or

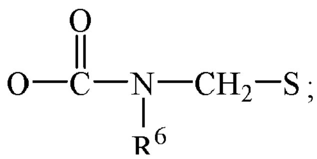

wherein $R^6$ is H, optionally substituted alkyl, optionally substituted aryl, optionally substituted heteroaryl, optionally substituted arylalkyl, or optionally substituted heteroarylalkyl; and
with the proviso that at least one of $R^1$ and $R^2$ is CN; $NO_2$;

optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted;
heteroaryl or heteroarylalkyl, each optionally substituted; or
$OR^9$ or $NR^9_2$ wherein each $R^9$ is independently H or optionally substituted alkyl, or both $R^9$ groups taken together with the nitrogen to which they are attached form a heterocyclic ring;

$SR^4$ wherein

$R^4$ is optionally substituted alkyl;
aryl or arylalkyl, each optionally substituted; or
heteroaryl or heteroarylalkyl, each optionally substituted;

wherein $R^1$ and $R^2$ may be joined to form a 3-8 membered ring; and
wherein any remaining $R^1$ and $R^2$ is H or is alkyl, arylalkyl or heteroarylalkyl, each optionally substituted; and any remaining $R^5$ is independently H or is alkyl, alkenylalkyl, alkynylalkyl, $(OCH_2CH_2)_p$ O-alkyl wherein p=1-1000, aryl, arylalkyl, heteroaryl or heteroarylalkyl, each optionally substituted.

11. The extended-release conjugate of embodiment 2 wherein L is a linker of the formula (3)

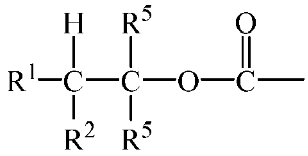

(3)

wherein at least one, or both $R^1$ and $R^2$ is independently CN; $NO_2$;

optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted;
heteroaryl or heteroarylalkyl, each optionally substituted; or $R^3$ is
$OR^9$ or $NR^9{}_2$ wherein each R is independently H or optionally substituted alkyl, or both $R^9$ groups taken together with the nitrogen to which they are attached form a heterocyclic ring;

$SR^4$ wherein

$R^4$ is optionally substituted alkyl;
aryl or arylalkyl, each optionally substituted; or
heteroaryl or heteroarylalkyl, each optionally substituted;

wherein $R^1$ and $R^2$ may be joined to form a 3-8 membered ring; and
wherein one and only one of $R^1$ and $R^2$ may be H or alkyl, arylalkyl or heteroarylalkyl, each optionally substituted; and
wherein one of $R^5$ is $(CH_2)_yZ^*$, $(CH_2CH_2O)_xCH_2CH_2Z^*$ or $(CH_2)_yNH\text{-}CO\text{-}(CH_2CH_2O)_xCH_2CH_2Z^*$, wherein x is 1-100, y = 1-6, and the other $R^5$ is H, alkyl, alkenylalkyl, alkynylalkyl, aryl, arylalkyl, heteroaryl or heteroarylalkyl, each optionally substituted; and
Z* indicates coupling to the insoluble matrix.

12. The extended-release conjugate of embodiment 11 wherein $R^1$ is CN or $R^3SO_2$, wherein $R^3$ is optionally substituted alkyl, optionally substituted aryl, optionally substituted heteroaryl, or $(R^9)_2N$, wherein each $R^9$ is independently H or optionally substituted alkyl;
wherein one of $R^5$ is $(CH_2)_yZ^*$, $(CH_2CH_2O)_xCH_2CH_2Z^*$ or $(CH_2)_yNH\text{-}CO\text{-}(CH_2CH_2O)_xCH_2CH_2Z^*$, wherein x is 1-100, y = 1-6, and the other $R^5$ is H or unsubstituted alkyl.

13. The extended-release conjugate of embodiment 12 wherein $R^1$ is CN or $CH_3SO_2$; $R^2$ is H; one $R^5$ is $(CH_2)_nZ^*$ wherein y is 5; and the other $R^5$ is H.

14. The extended-release conjugate of embodiment 6 wherein the linker-agonist peptide is covalently coupled to the crosslinkers.

15. A compound of formula (4)

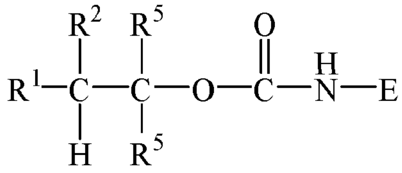 (4)

wherein E is a GLP-1 agonist that shows less than 10% chemical degradation after 1 month at pH 7.4, 37°C wherein at least one, or both $R^1$ and $R^2$ is independently CN; $NO_2$;

- optionally substituted aryl;
- optionally substituted heteroaryl;
- optionally substituted alkenyl;
- optionally substituted alkynyl;
- $COR^3$ or $SOR^3$ or $SO_2R^3$ wherein
  - $R^3$ is H or optionally substituted alkyl;
  - aryl or arylalkyl, each optionally substituted;
  - heteroaryl or heteroarylalkyl, each optionally substituted; or $R^3$ is
  - $OR^9$ or $NR^9_2$ wherein each R is independently H or optionally substituted alkyl, or both $R^9$ groups taken together with the nitrogen to which they are attached form a heterocyclic ring;
- $SR^4$ wherein
  - $R^4$ is optionally substituted alkyl;
  - aryl or arylalkyl, each optionally substituted; or
  - heteroaryl or heteroarylalkyl, each optionally substituted;

wherein $R^1$ and $R^2$ may be joined to form a 3-8 membered ring; and
wherein one and only one of $R^1$ and $R^2$ may be H or alkyl, arylalkyl or heteroarylalkyl, each optionally substituted; and
wherein one of $R^5$ is $(CH_2)_yZ$, $(CH_2CH_2O)_xCH_2CH_2Z$ or $(CH_2)_yNH\text{-}CO\text{-}(CH_2CH_2O)_xCH_2CH_2Z$, wherein x is 1-100, y = 1-6, and the other $R^5$ is H, alkyl, alkenylalkyl, alkynylalkyl, aryl, arylalkyl, heteroaryl or heteroarylalkyl, each optionally substituted; and
Z is a functional group for coupling to said insoluble matrix.

16. The compound of embodiment 15 wherein said functional group comprises $N_3$, $NH_2$, $NH\text{-}CO_2{}^tBu$, SH, $S^tBu$, maleimide, $CO_2H$, $CO_2{}^tBu$, 1,3-diene, cyclopentadiene, furan, alkyne, cyclooctyne, acrylate, aminooxy, keto or acrylamide.

17. The compound of embodiment 15 wherein $R^1$ is CN or $R^3SO_2$, wherein $R^3$ is optionally substituted alkyl, optionally substituted aryl, optionally substituted heteroaryl, or $(R^9)_2N$, wherein each $R^9$ is independently H or optionally substituted alkyl;
wherein one of $R^5$ is $(CH_2)_yZ$, $(CH_2CH_2O)_xCH_2CH_2Z$ or $(CH_2)_yNH\text{-}CO\text{-}(CH_2CH_2O)_xCH_2CH_2Z$, wherein x is 1-100, y = 1-6, and the other $R^5$ is H or unsubstituted alkyl.

18. The compound of embodiment 17 wherein $R^1$ is CN or $CH_3SO_2$; $R^2$ is H; one $R^5$ is $(CH_2)_yZ$ wherein y is 5; and the other $R^5$ is H.

19. The compound of any of embodiments 15-18 wherein E is a stabilized exendin comprising an amino acid substitution at the position corresponding to N28 in the native exendin sequence, wherein the native exendin consists of SEQ ID NO: 1 or SEQ ID NO:7.

20. The compound of embodiment 19 wherein E is N29D, N28A, N28K or N28Q substituted SEQ ID NO: 1 or SEQ ID NO:7.

21. The compound of embodiment 20 wherein E is SEQ ID NO:2.

22. A protocol for administering a GLP-1 agonist which comprises administering to a subject having a condition benefited by a GLP-1 agonist the conjugate of any of embodiments 1-5 one dosage per one to three months.

23. A peptide of the formula:
HGEGTFTSDL SKQMEEEAVR LFIEWLKQGG PSSGAPPPS-$NH_2$ (SEQ ID NO:2) ([N28Q]exenatide) or a pharmaceutically acceptable salt thereof.

24. A pharmaceutical composition comprising as active ingredient the peptide or salt of embodiment 23.

25. A protocol for administering a GLP-1 agonist which comprises administering to a subject having a condition benefited by a GLP-1 agonist the peptide or salt of embodiment 23 or the pharmaceutical composition of embodiment 24.

## Claims

1. An extended-release conjugate of a GLP-1 agonist peptide comprising an insoluble matrix with a multiplicity of covalently attached linker-GLP-1 agonist peptides each comprising a linker covalently bound to a GLP-1 agonist peptide, wherein the linkers cleave under physiological conditions of pH and temperature to release free GLP-1 agonist peptides and wherein the GLP-1 agonist peptide of said linker-GLP-1 agonist peptide shows a degradation of less than 10% over one month under physiological conditions of pH and temperature, wherein the GLP-1 agonist peptide is SEQ ID NO: 2 or SEQ ID NO: 8.

2. The extended-release conjugate of claim 1, having the formula

$$M\text{-}(L\text{-}E)_x$$

wherein M is an insoluble matrix; L is a cleavable linker having a half-life of cleavage at 37°C, pH 7.4, of between 320 and 2400 hours; and E is a GLP-1 agonist peptide that shows less than 10% chemical degradation after 1 month at pH 7.4, 37°C and x is an integer representing the number of L-E units required to provide a concentration of 1-1000 mg GLP-1 agonist peptide per mL of matrix.

3. The extended-release conjugate of Claim 1 or 2 wherein the insoluble matrix is a biodegradable crosslinked hydrogel comprising polymers coupled by crosslinkers.

4. The extended-release conjugate of Claim 3 wherein the hydrogel is a biodegradable crosslinked poly(ethylene glycol).

5. The extended-release conjugate of Claim 3 wherein the hydrogel is in the form of microspheres.

6. The extended-release conjugate of Claim 3, wherein the polymers comprising the hydrogel are crosslinked by crosslinkers that are cleavable by beta elimination.

7. The extended-release conjugate of Claim 6, wherein: said crosslinkers contained in the hydrogel are of formula (1)

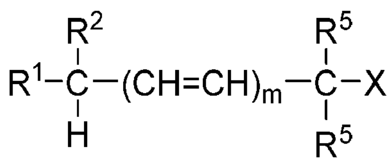

(1)

wherein m is 0 or 1; and
wherein X comprises the residue of a functional group coupled to a polymer contained in the hydrogel and one of $R^1$, $R^2$ and $R^5$ is coupled to a polymer contained in the hydrogel,

with the proviso that at least one of $R^1$ and $R^2$ is CN; $NO_2$;
optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted;
heteroaryl or heteroarylalkyl, each optionally substituted; or

$OR^9$ or $NR^9_2$ wherein each $R^9$ is independently H or optionally substituted alkyl, or both $R^9$ groups taken together with the nitrogen to which they are attached form a heterocyclic ring;
$SR^4$ wherein

$R^4$ is optionally substituted alkyl;
aryl or arylalkyl, each optionally substituted; or
heteroaryl or heteroarylalkyl, each optionally substituted;

wherein $R^1$ and $R^2$ may be joined to form a 3-8 membered ring; and
wherein any remaining $R^1$ and $R^2$ is H or is alkyl, arylalkyl or heteroarylalkyl, each optionally substituted; and any remaining $R^5$ is independently H or is alkyl, alkenylalkyl, alkynylalkyl, $(OCH_2CH_2)_pO$-alkyl wherein p=1-1000, aryl, arylalkyl, heteroaryl or heteroarylalkyl, each optionally substituted; or
said crosslinkers when contained in the hydrogel are of formula (2)

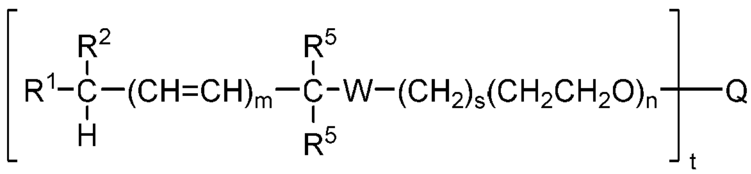

(2)

wherein two of $R^1$, $R^2$ and $R^5$ are coupled to a polymer contained in the hydrogel;
m is 0-1;
n is 1-1000;
s is 0-2;
t is 2,4, 8, 16 or 32;
Q is a core group having the valency t;
W is O(C=O)O, O(C=O)NH, O(C=O)S,

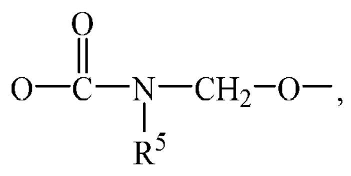

or

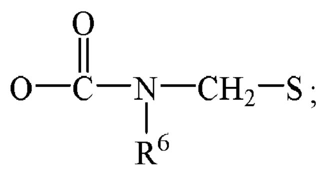

wherein $R^6$ is H, optionally substituted alkyl, optionally substituted aryl, optionally substituted heteroaryl, optionally substituted arylalkyl, or optionally substituted heteroarylalkyl; and
with the proviso that at least one of $R^1$ and $R^2$ is CN; $NO_2$;

optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted;
heteroaryl or heteroarylalkyl, each optionally substituted; or
$OR^9$ or $NR^9_2$ wherein each $R^9$ is independently H or optionally substituted alkyl, or both $R^9$ groups taken together with the nitrogen to which they are attached form a heterocyclic ring;

$SR^4$ wherein

$R^4$ is optionally substituted alkyl;
aryl or arylalkyl, each optionally substituted; or
heteroaryl or heteroarylalkyl, each optionally substituted;

wherein $R^1$ and $R^2$ may be joined to form a 3-8 membered ring; and
wherein any remaining $R^1$ and $R^2$ is H or is alkyl, arylalkyl or heteroarylalkyl, each optionally substituted; and any remaining $R^5$ is independently H or is alkyl, alkenylalkyl, alkynylalkyl, $(OCH_2CH_2)_p$ O-alkyl wherein p=1-1000, aryl, arylalkyl, heteroaryl or heteroarylalkyl, each optionally substituted.

**8.** The extended-release conjugate of claim 2, wherein L is a linker of the formula (3)

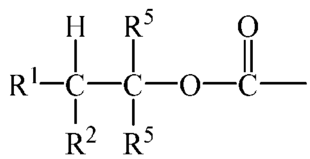

(3)

wherein at least one, or both $R^1$ and $R^2$ is independently CN; $NO_2$;

optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted; heteroaryl or heteroarylalkyl, each optionally substituted; or $R^3$ is $OR^9$ or $NR^9_2$ wherein each R is independently H or optionally substituted alkyl, or both $R^9$ groups taken together with the nitrogen to which they are attached form a heterocyclic ring;

$SR^4$ wherein

$R^4$ is optionally substituted alkyl;

aryl or arylalkyl, each optionally substituted; or
heteroaryl or heteroarylalkyl, each optionally substituted;

wherein $R^1$ and $R^2$ may be joined to form a 3-8 membered ring; and
wherein one and only one of $R^1$ and $R^2$ may be H or alkyl, arylalkyl or heteroarylalkyl, each optionally substituted; and
wherein one of $R^5$ is $(CH_2)_yZ^*$, $(CH_2CH_2O)_xCH_2CH_2Z^*$ or $(CH_2)_yNH\text{-}CO\text{-}(CH_2CH_2O)_xCH_2CH_2Z^*$, wherein x is 1-100, y = 1-6, and the other $R^5$ is H, alkyl, alkenylalkyl, alkynylalkyl, aryl, arylalkyl, heteroaryl or heteroarylalkyl, each optionally substituted; and
Z* indicates a functional group for coupling to the insoluble matrix.

**9.** The extended-release conjugate of Claim 8 wherein $R^1$ is CN or $R^3SO_2$, wherein $R^3$ is optionally substituted alkyl, optionally substituted aryl, optionally substituted heteroaryl, or $(R^9)_2N$, wherein each $R^9$ is independently H or optionally substituted alkyl;
wherein one of $R^5$ is $(CH_2)_yZ^*$, $(CH_2CH_2O)_xCH_2CH_2Z^*$ or $(CH_2)_yNH\text{-}CO\text{-}(CH_2CH_2O)_xCH_2CH_2Z^*$, wherein x is 1-100, y = 1-6, and the other $R^5$ is H or unsubstituted alkyl.

**10.** The extended-release conjugate of Claim 9 wherein $R^1$ is CN or $CH_3SO_2$; $R^2$ is H; one $R^5$ is $(CH_2)_nZ^*$ wherein y is 5; and the other $R^5$ is H.

**11.** The extended-release conjugate of Claim 3 wherein the linker-agonist peptides are covalently coupled to the crosslinkers.

**12.** A compound of formula (4)

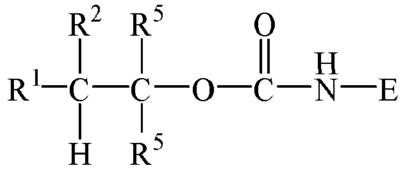

(4)

wherein E is a GLP-1 agonist peptide that shows less than 10% chemical degradation after 1 month at pH 7.4, 37°C
wherein at least one, or both $R^1$ and $R^2$ is independently CN; $NO_2$;

optionally substituted aryl;
optionally substituted heteroaryl;
optionally substituted alkenyl;
optionally substituted alkynyl;
$COR^3$ or $SOR^3$ or $SO_2R^3$ wherein

$R^3$ is H or optionally substituted alkyl;
aryl or arylalkyl, each optionally substituted;
heteroaryl or heteroarylalkyl, each optionally substituted; or $R^3$ is
$OR^9$ or $NR^9_2$ wherein each R is independently H or optionally substituted alkyl, or both $R^9$ groups taken together with the nitrogen to which they are attached form a heterocyclic ring;

$SR^4$ wherein

$R^4$ is optionally substituted alkyl;
aryl or arylalkyl, each optionally substituted; or
heteroaryl or heteroarylalkyl, each optionally substituted;

wherein $R^1$ and $R^2$ may be joined to form a 3-8 membered ring; and
wherein one and only one of $R^1$ and $R^2$ may be H or alkyl, arylalkyl or heteroarylalkyl, each optionally substituted; and
wherein one of $R^5$ is $(CH_2)_yZ$, $(CH_2CH_2O)_xCH_2CH_2Z$ or $(CH_2)_yNH\text{-}CO\text{-}(CH_2CH_2O)_xCH_2CH_2Z$, wherein x is

1-100, y = 1-6, and the other $R^5$ is H, alkyl, alkenylalkyl, alkynylalkyl, aryl, arylalkyl, heteroaryl or heteroarylalkyl, each optionally substituted; and
Z is a functional group for coupling to said insoluble matrix.

**13.** The compound of Claim 12, wherein E is a stabilized exendin comprising an amino acid substitution at the position corresponding to N28 in the native exendin sequence, wherein the native exendin consists of SEQ ID NO: 1 or SEQ ID NO:7.

**14.** The compound of Claim 13 wherein E is N29D, N28A, N28K or N28Q substituted SEQ ID NO: 1 or SEQ ID NO:7.

**15.** A GLP-1 agonist peptide conjugate of Claim 1 for use in a method of treating a condition benefited by the GLP-1 agonist peptide wherein one dosage per one to three months is administered to a subject having a condition benefited by the GLP-1 agonist conjugate.

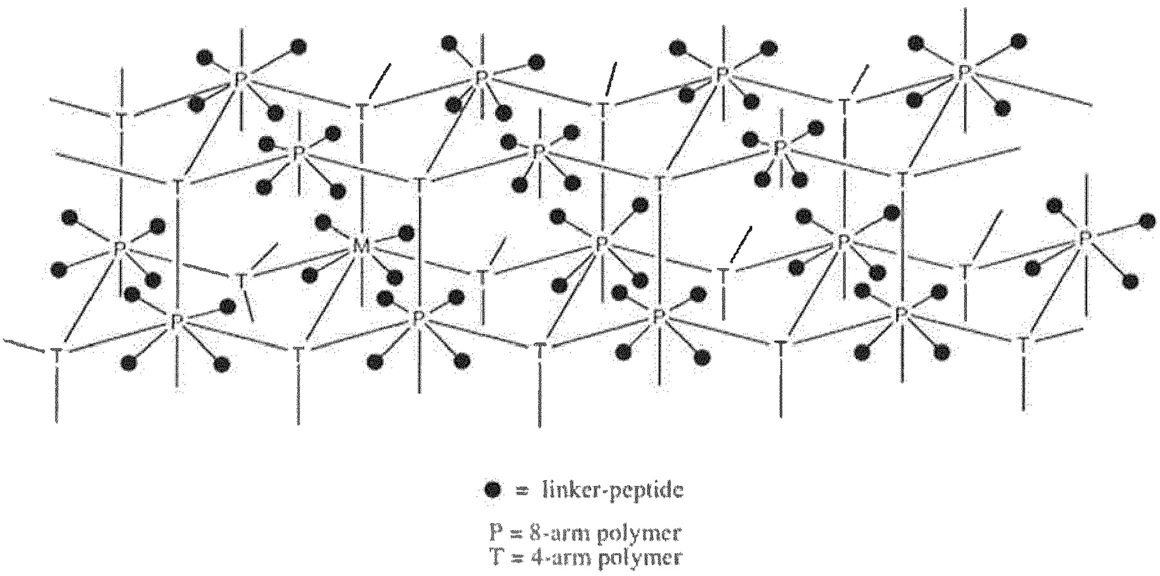


Figure 1A

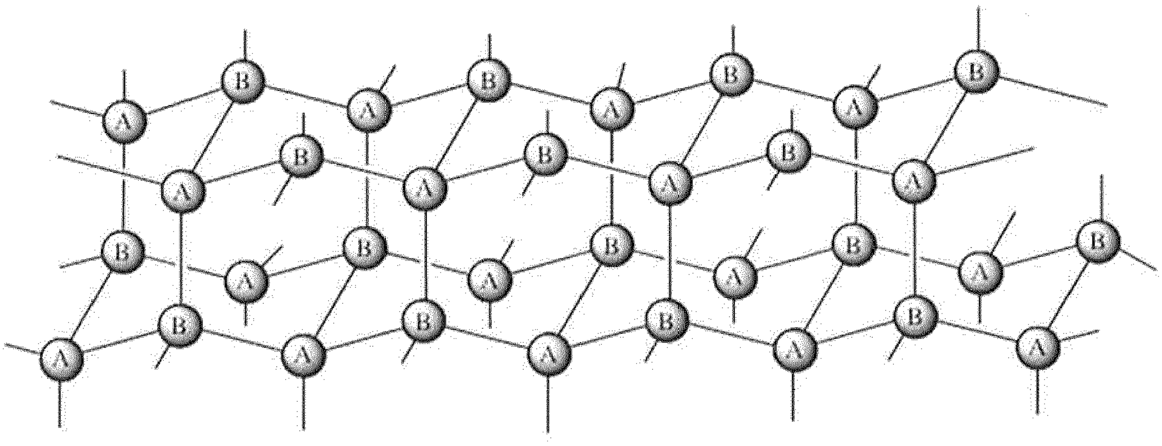


Figure 1B

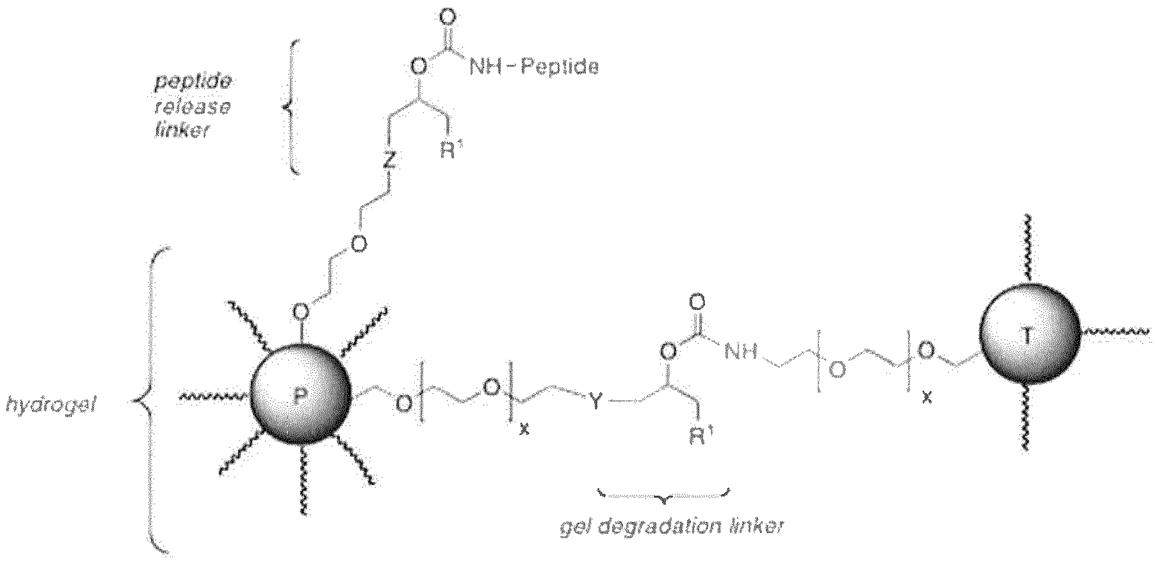


Y,Z = connecting functionality

each P comprises N linker-peptides and (8-N) crosslinks to T

Figure 2

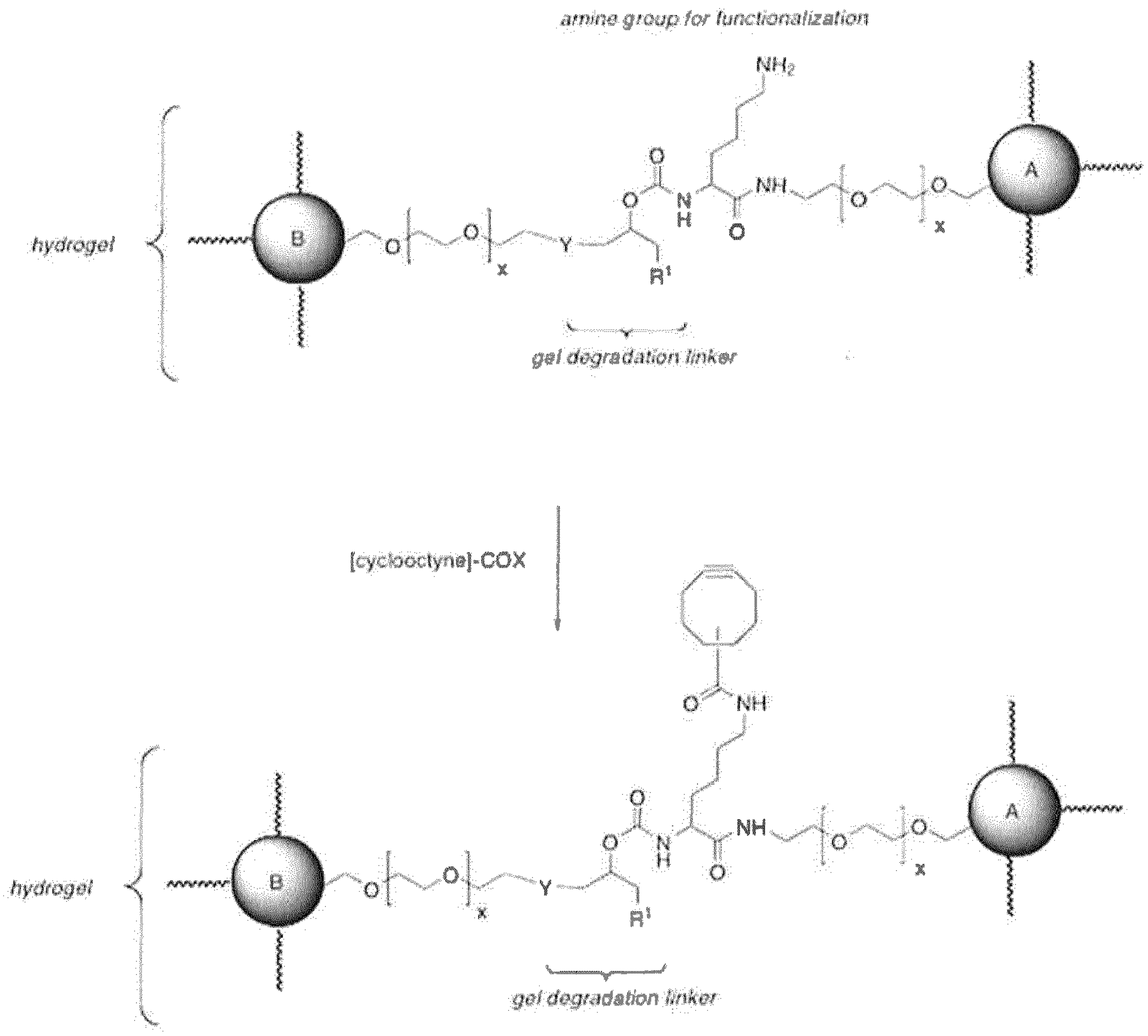
amine group for functionalization
hydrogel
gel degradation linker
[cyclooctyne]-COX
hydrogel
gel degradation linker

Figure 3

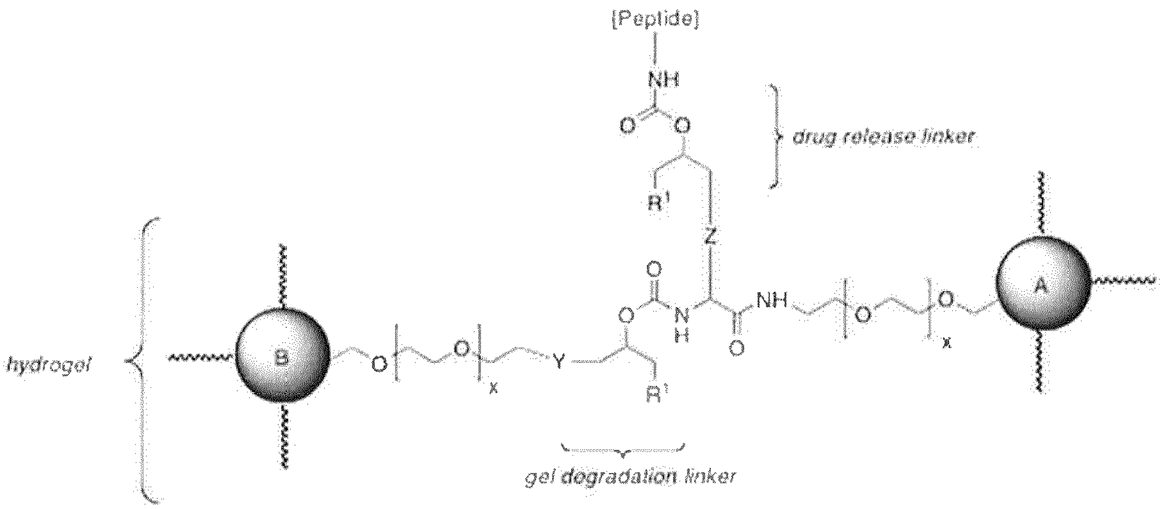


Y,Z = connecting functionality

Figure 4

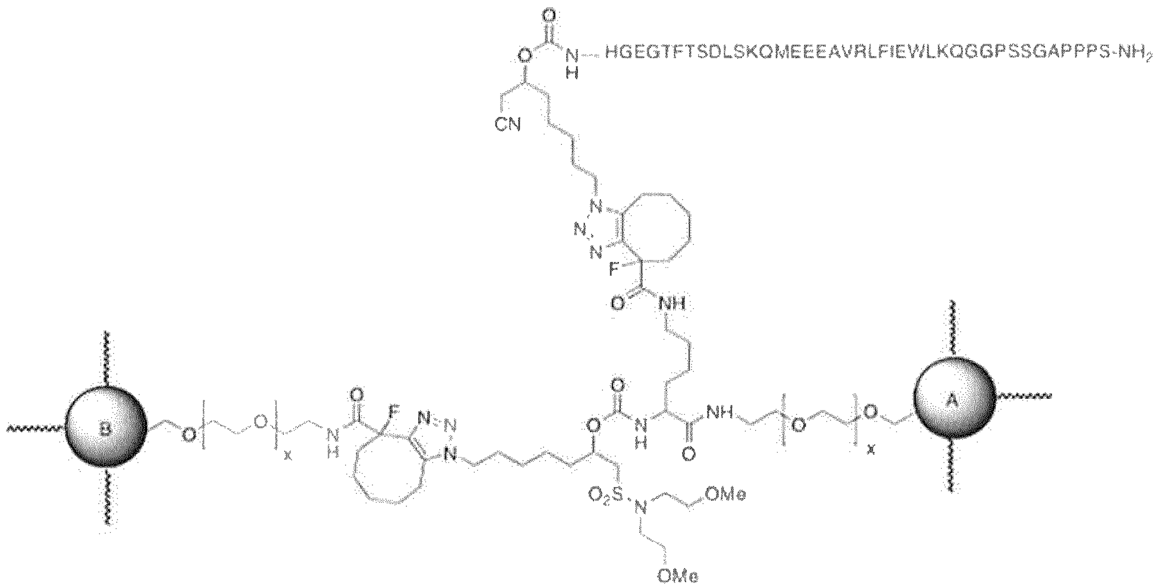

Figure 5

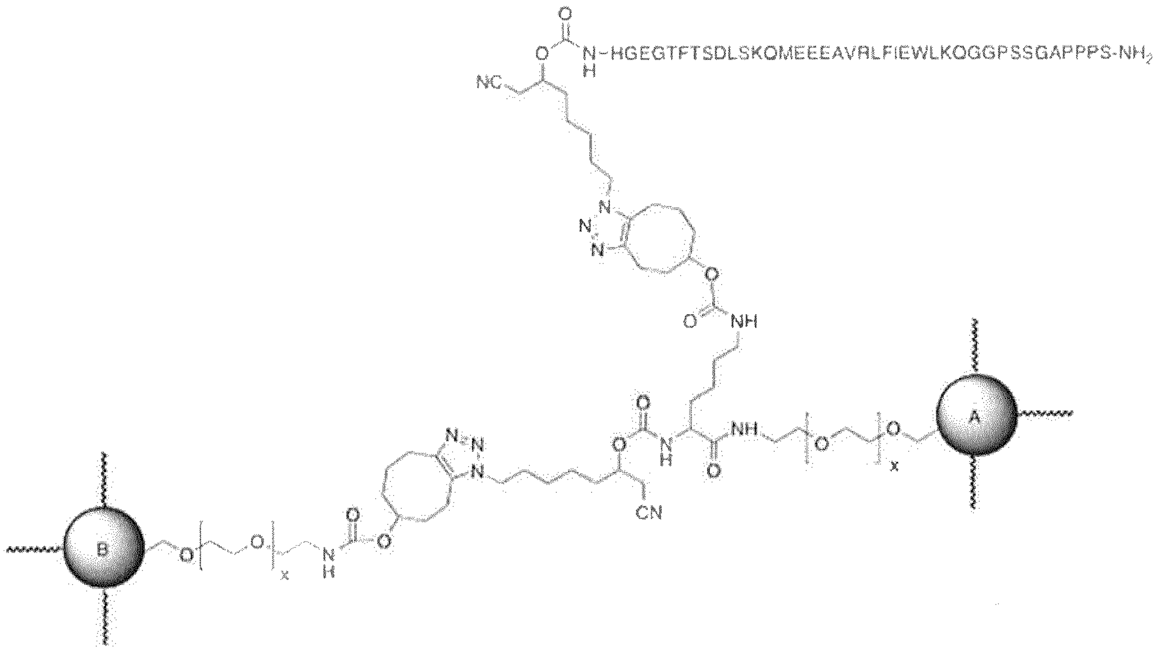

Figure 6

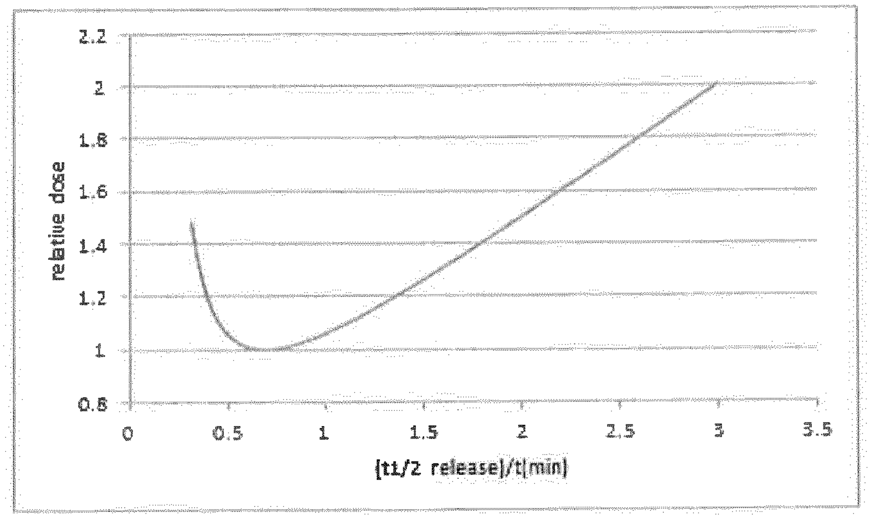
relative dose
2.2
2
1.8
1.6
1.4
1.2
1
0.8
0
0.5
1
1.5
2
2.5
3
3.5
(t1/2 release)/t(min)

A

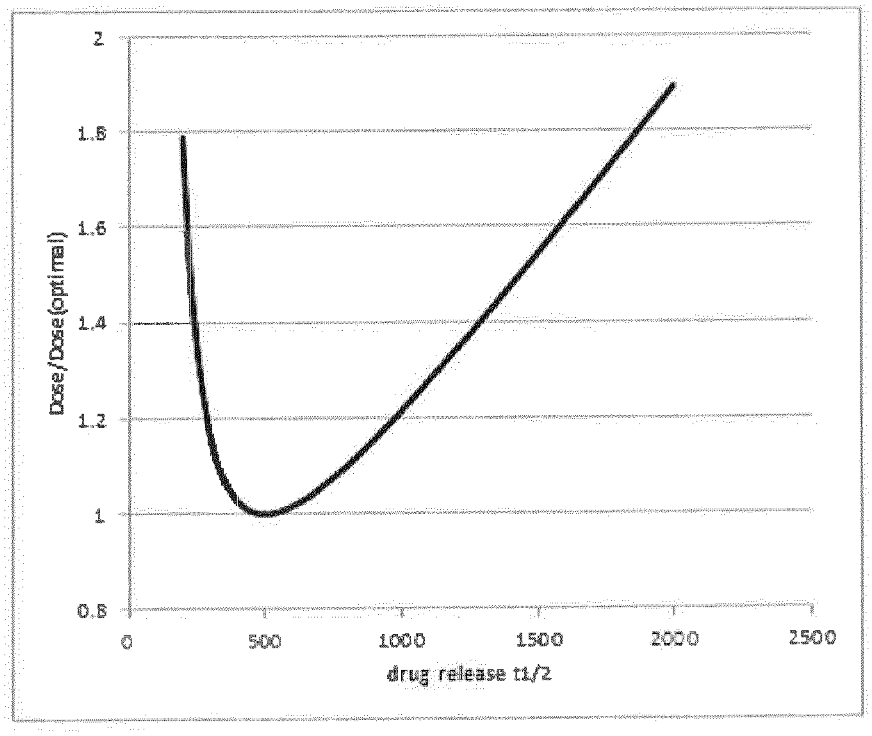
Dose/Dose(optimal)
2
1.8
1.6
1.4
1.2
1
0.8
0
500
1000
1500
2000
2500
drug release t1/2

B

**Figure 7**

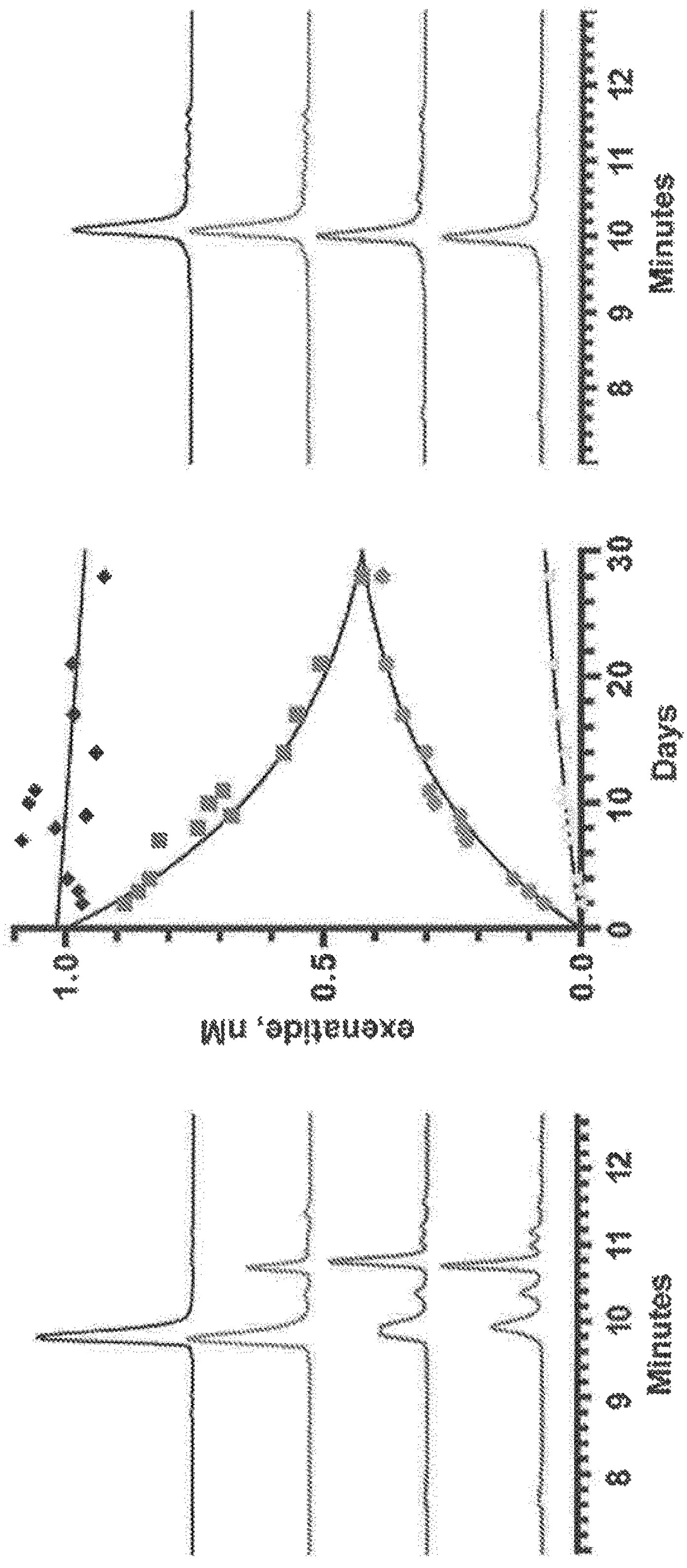


A B C

Figure 8

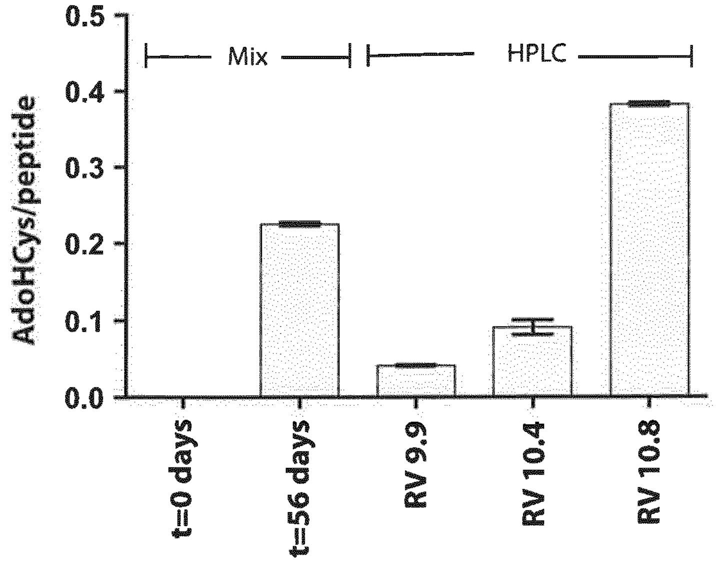


Figure 9

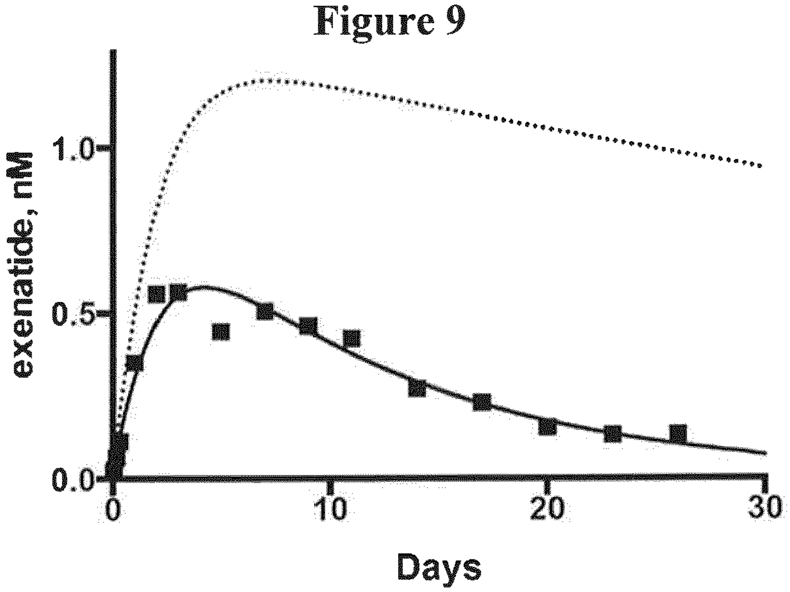


Figure 10

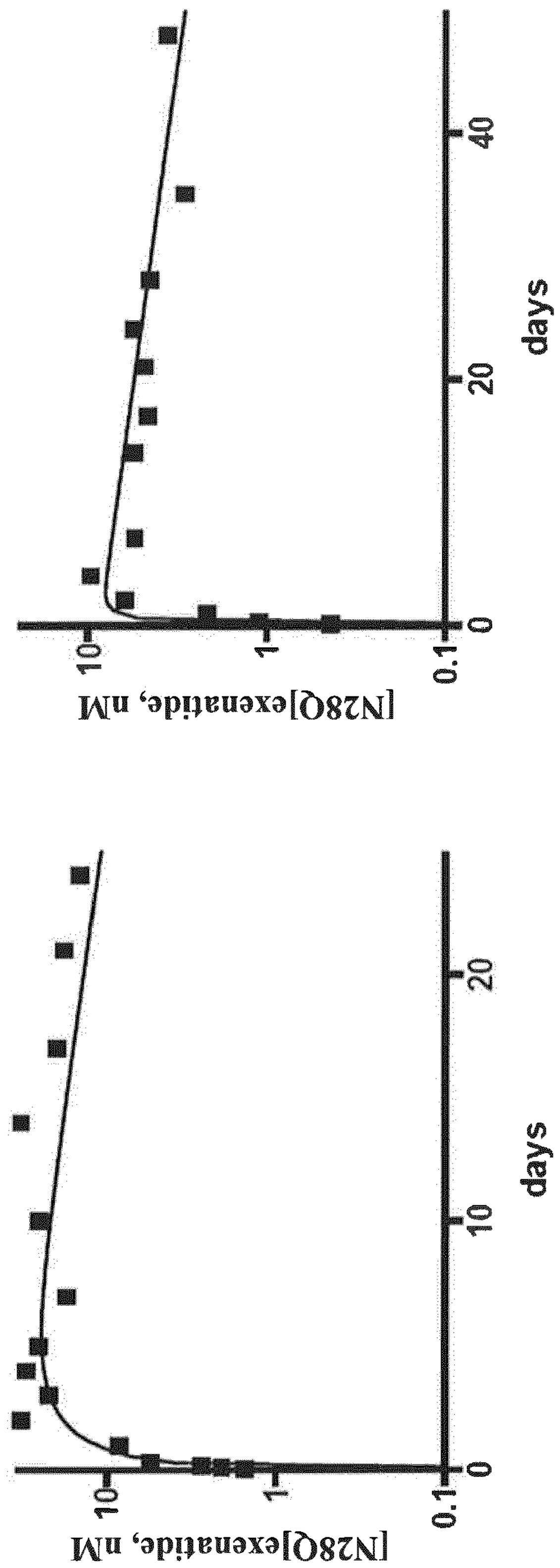
[N28Q]exenatide, nM
10
1
0.1
0
10
20
days
[N28Q]exenatide, nM
10
1
0.1
0
20
40
days

A

B

Figure 11

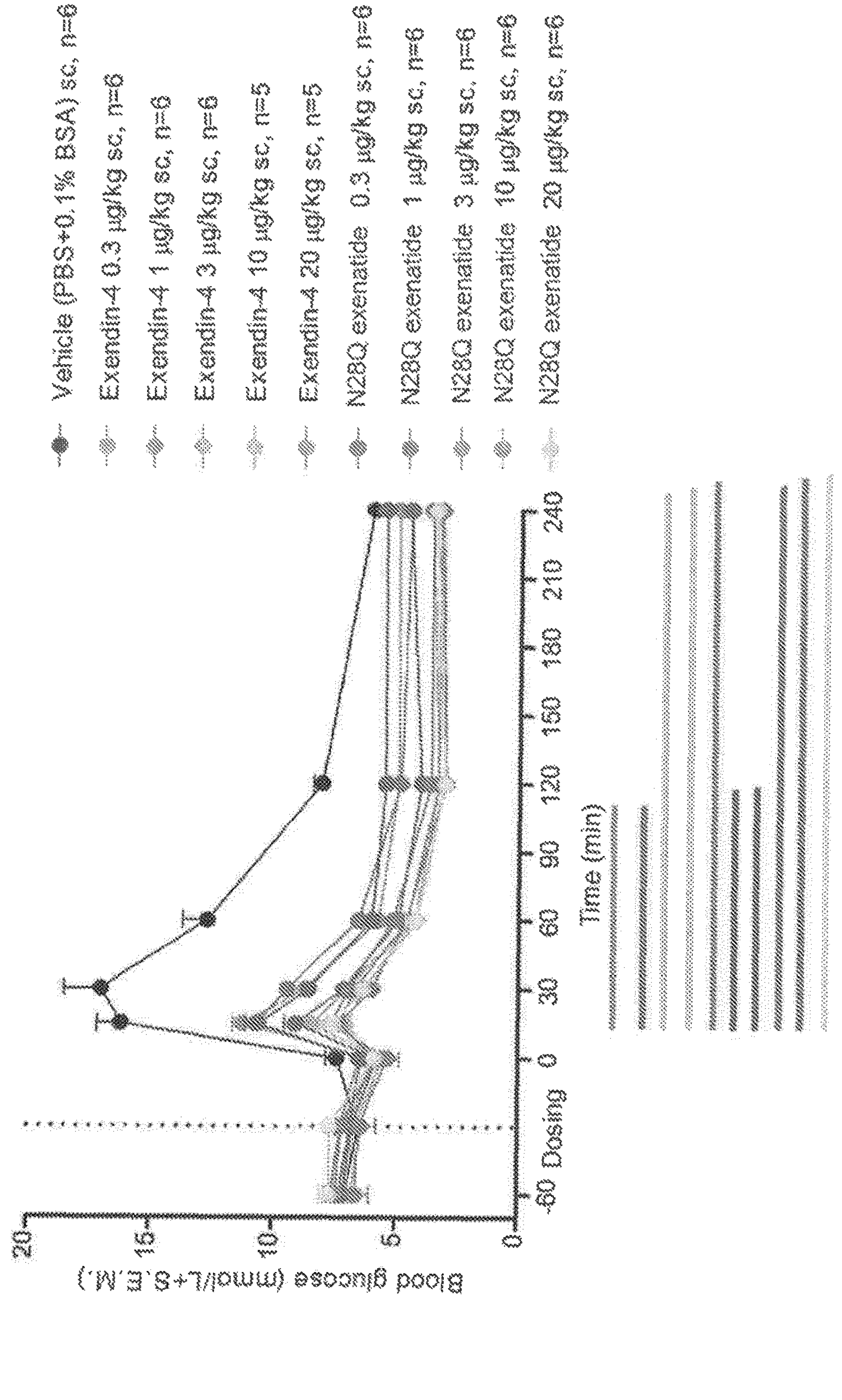
OGTT
Blood glucose (mmol/L+S.E.M.)
20
15
10
5
0
-60 Dosing 0 30 60 90 120 150 180 210 240
Time (min)
Vehicle (PBS+0.1% BSA) sc, n=6
Exendin-4 0.3 µg/kg sc, n=6
Exendin-4 1 µg/kg sc, n=6
Exendin-4 3 µg/kg sc, n=6
Exendin-4 10 µg/kg sc, n=5
Exendin-4 20 µg/kg sc, n=5
N28Q exenatide 0.3 µg/kg sc, n=6
N28Q exenatide 1 µg/kg sc, n=6
N28Q exenatide 3 µg/kg sc, n=6
N28Q exenatide 10 µg/kg sc, n=6
N28Q exenatide 20 µg/kg sc, n=6

Figure 12

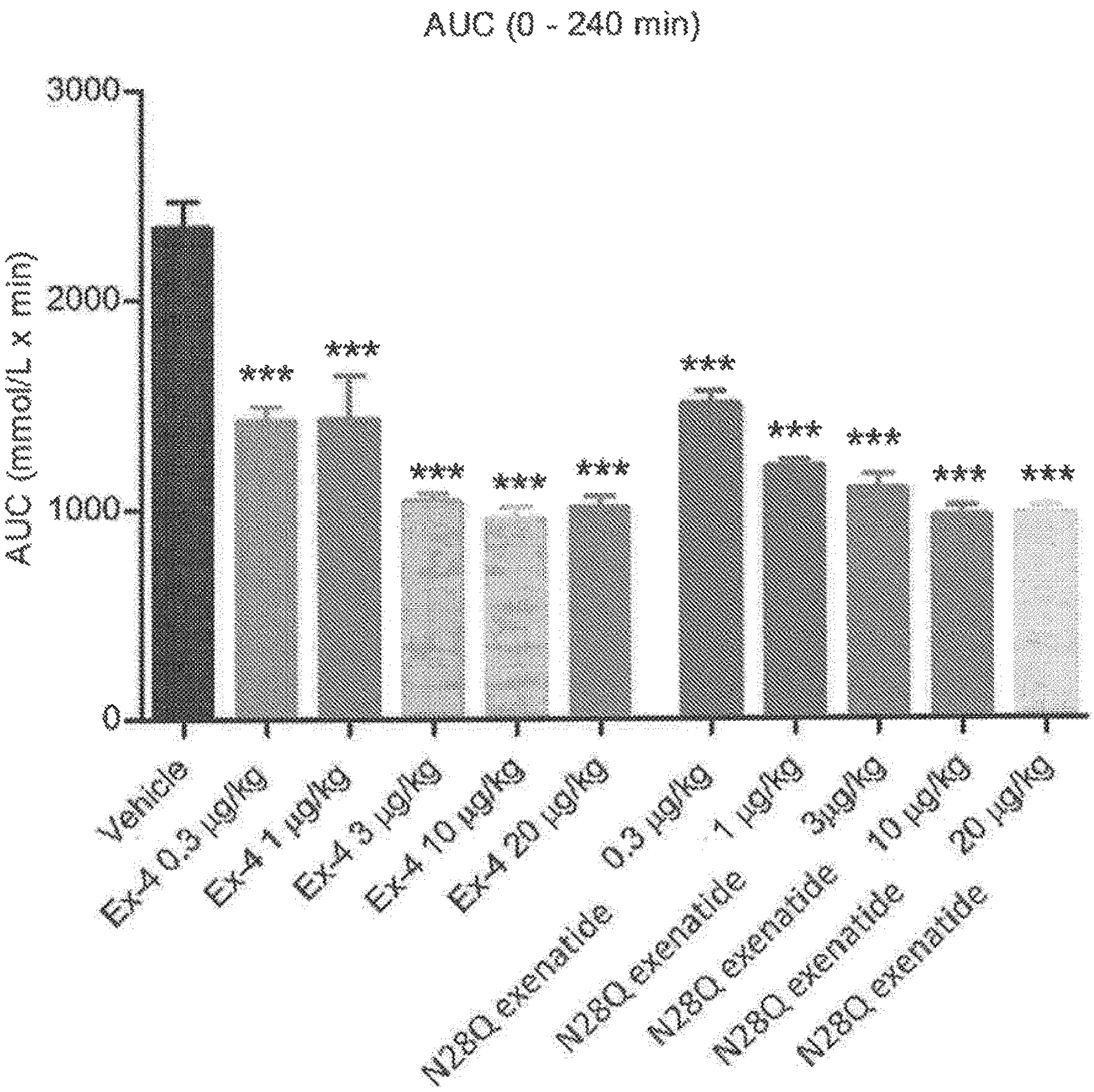
AUC (0 - 240 min)
AUC (mmol/L x min)
3000
2000
1000
0
***
Vehicle
Ex-4 0.3 µg/kg
Ex-4 1 µg/kg
Ex-4 3 µg/kg
Ex-4 10 µg/kg
Ex-4 20 µg/kg
N28Q exenatide 0.3 µg/kg
N28Q exenatide 1 µg/kg
N28Q exenatide 3µg/kg
N28Q exenatide 10 µg/kg
N28Q exenatide 20 µg/kg

Figure 13

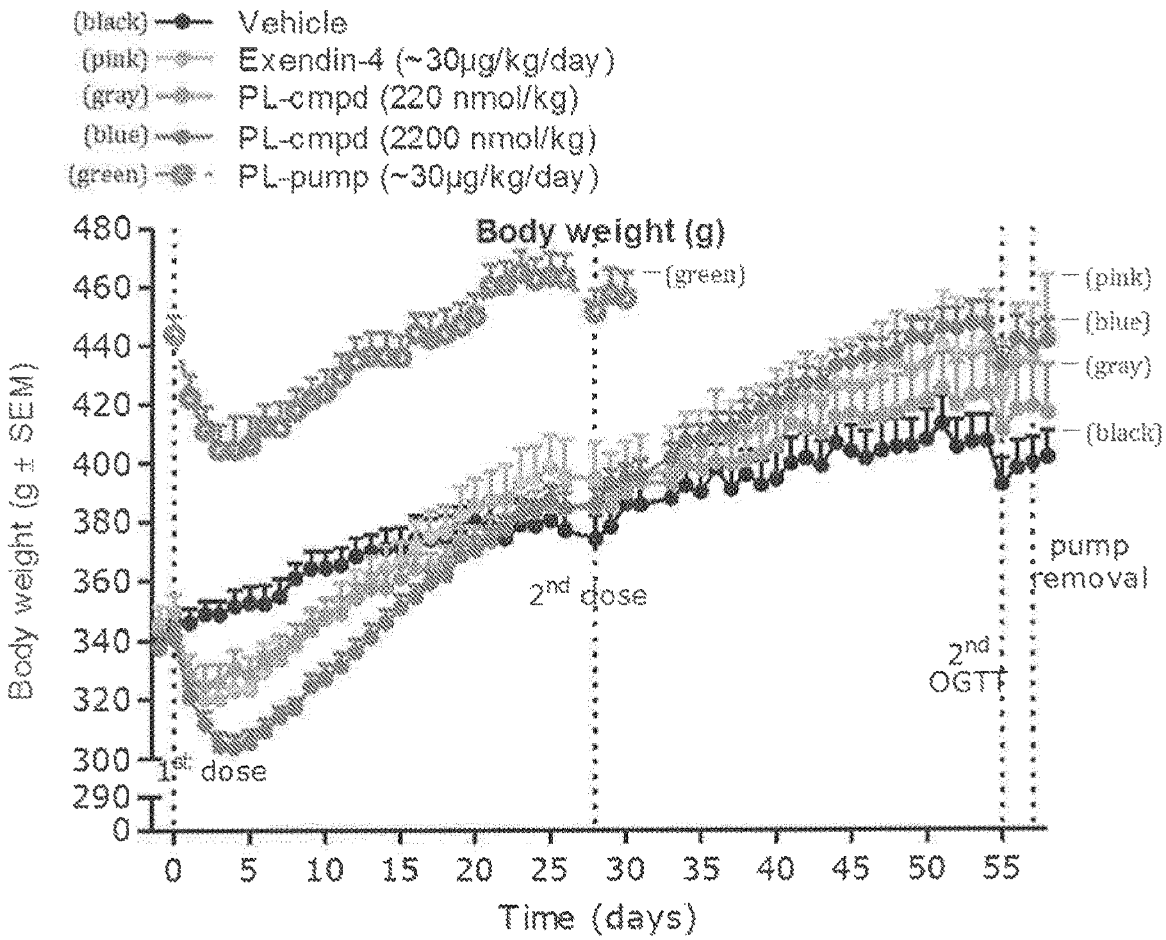
(black) Vehicle
(pink) Exendin-4 (~30μg/kg/day)
(gray) PL-cmpd (220 nmol/kg)
(blue) PL-cmpd (2200 nmol/kg)
(green) PL-pump (~30μg/kg/day)
Body weight (g)
Body weight (g ± SEM)
480
460
440
420
400
380
360
340
320
300
290
0
(green)
(pink)
(blue)
(gray)
(black)
1st dose
2nd dose
2nd OGTT
pump removal
0
5
10
15
20
25
30
35
40
45
50
55
Time (days)

Figure 14A

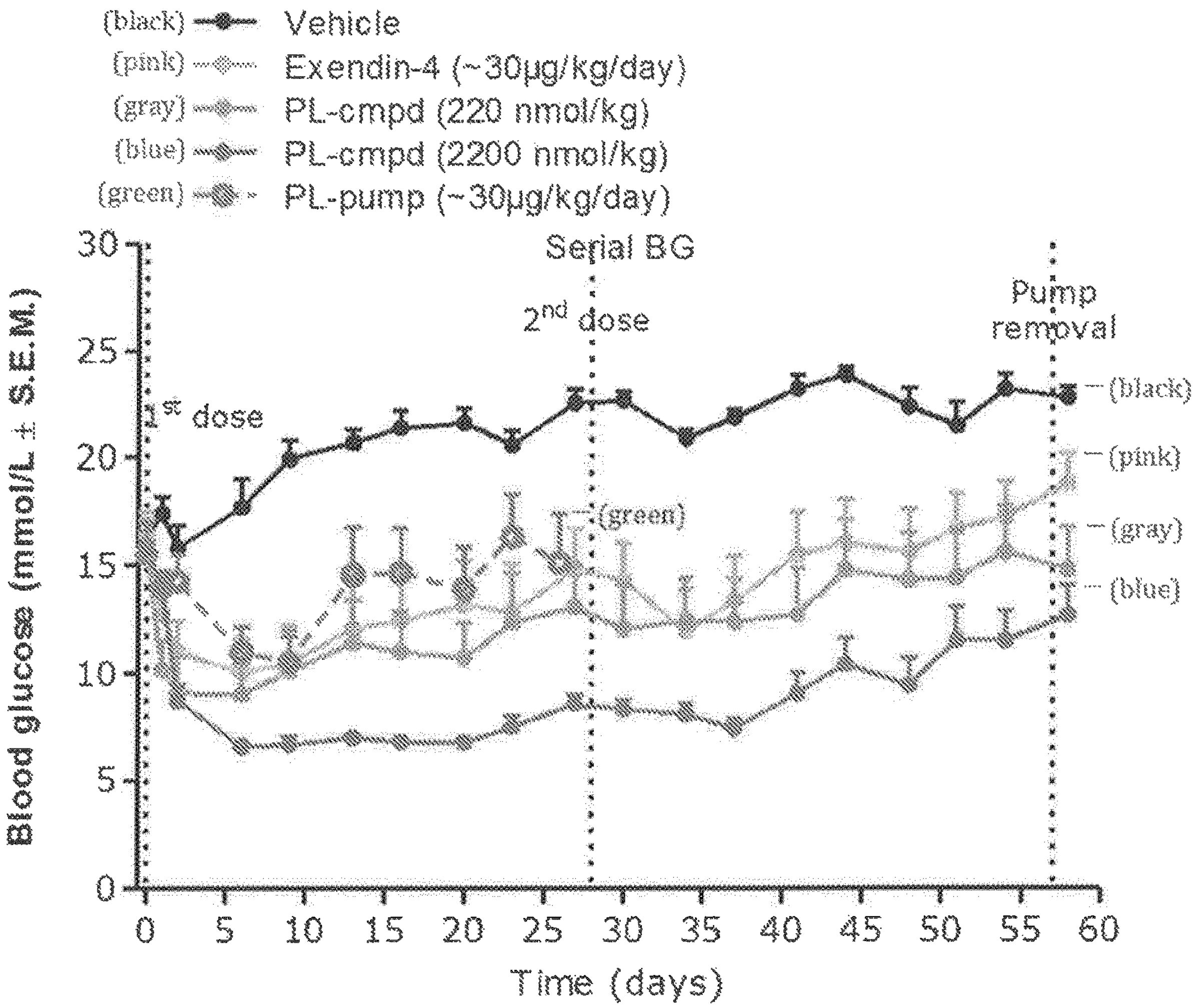
(black) Vehicle
(pink) Exendin-4 (~30μg/kg/day)
(gray) PL-cmpd (220 nmol/kg)
(blue) PL-cmpd (2200 nmol/kg)
(green) PL-pump (~30μg/kg/day)
Blood glucose (mmol/L ± S.E.M.)
Time (days)
1st dose
Serial BG
2nd dose
Pump removal
(green)
(black)
(pink)
(gray)
(blue)
0
5
10
15
20
25
30
0
5
10
15
20
25
30
35
40
45
50
55
60

Figure 14B

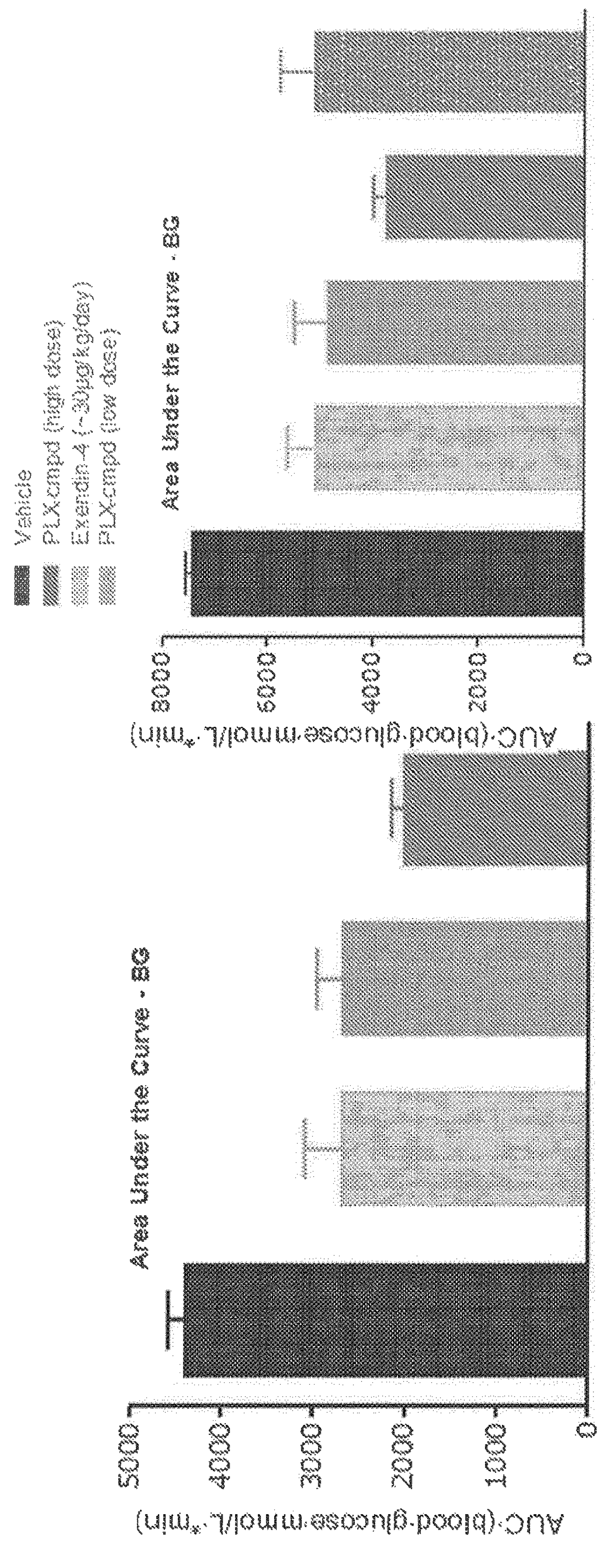
Vehicle
PLX.cmpd (high dose)
Exendin-4 (~30μg/kg/day)
PLX.cmpd (low dose)
Area Under the Curve - BG
AUC (blood glucose mmol/L*min)
8000
6000
4000
2000
0
Area Under the Curve - BG
AUC (blood glucose mmol/L*min)
5000
4000
3000
2000
1000
0

Figure 14C

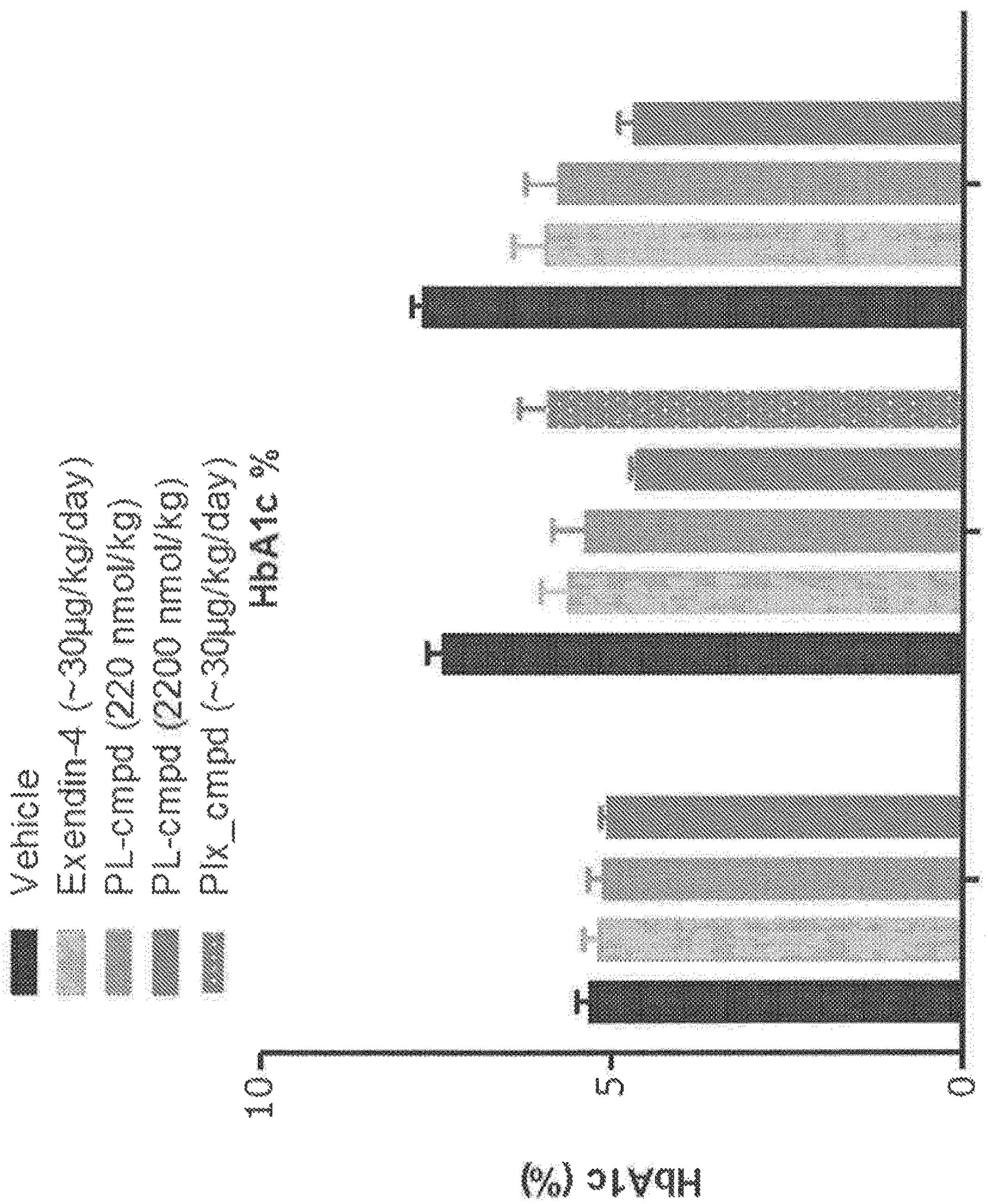
HbA1c %
Vehicle
Exendin-4 (~30μg/kg/day)
PL-cmpd (220 nmol/kg)
PL-cmpd (2200 nmol/kg)
Plx_cmpd (~30μg/kg/day)
HbA1c (%)
10
5
0
Day -1
Day 26
Day 55

Figure 14D

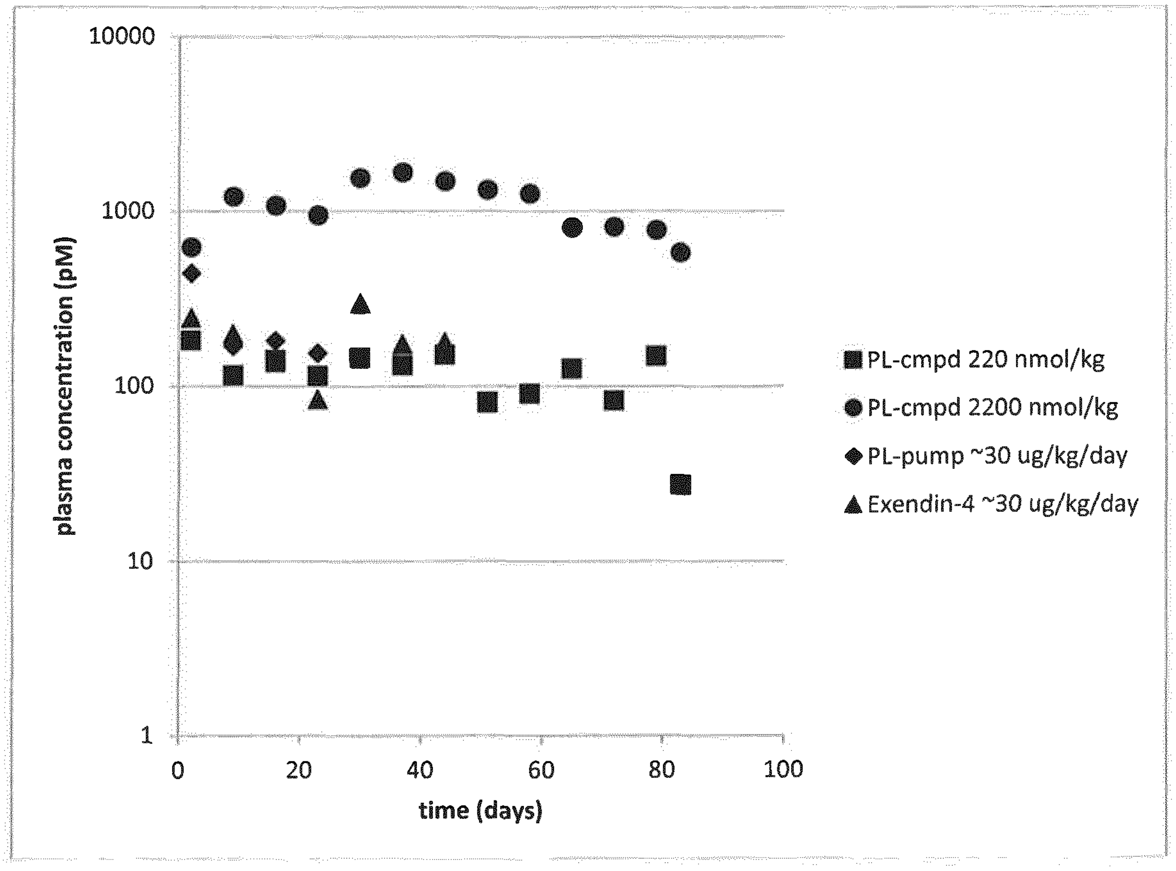


**FIGURE 14E**. Plasma concentrations of free peptides in pM.

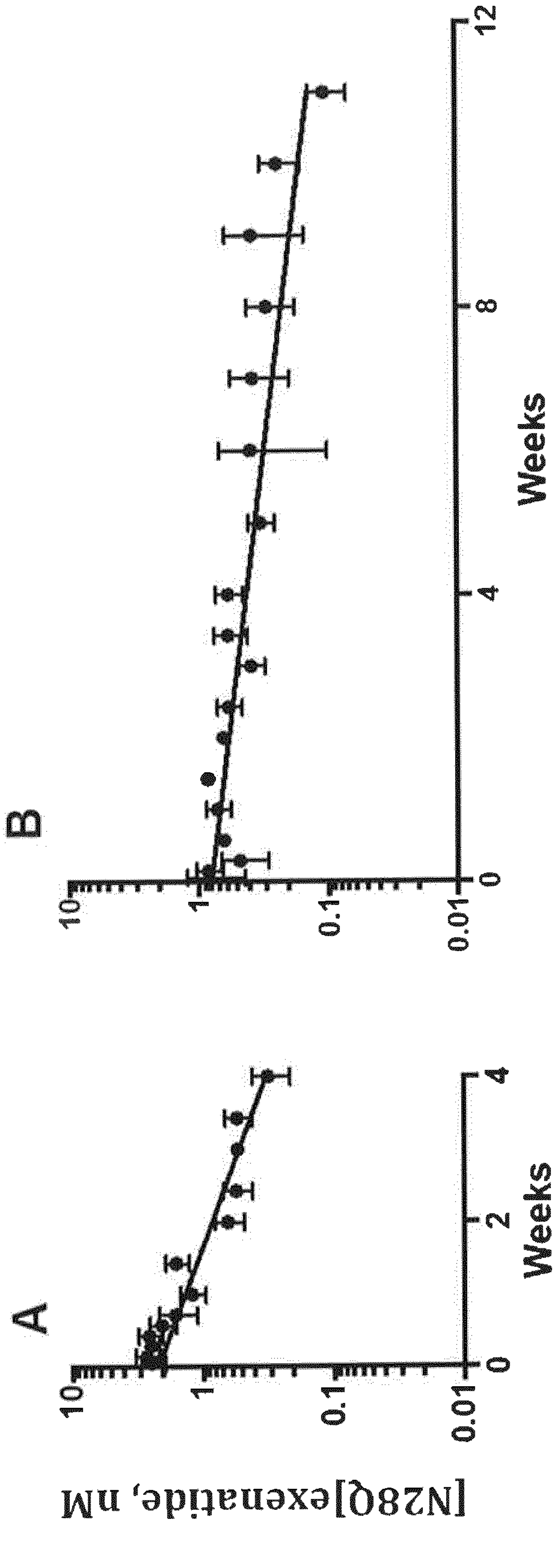


FIGURE 15

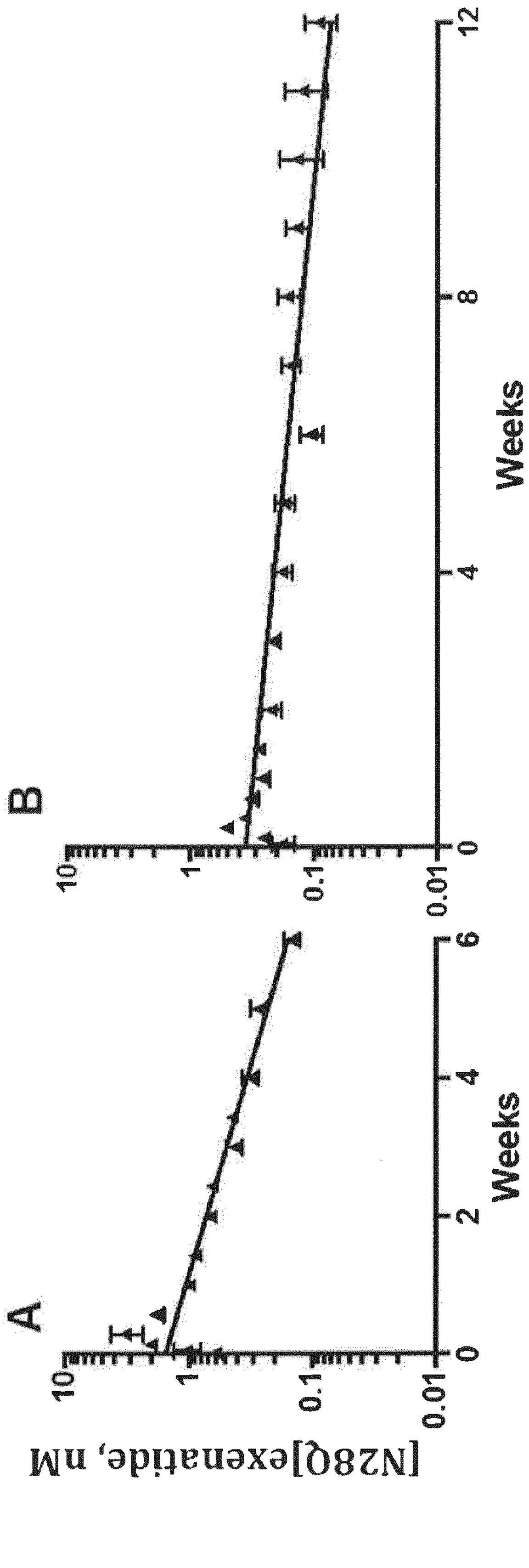


FIGURE 16

Europäisches Patentamt
European Patent Office
Office européen des brevets

# EUROPEAN SEARCH REPORT

Application Number
EP 21 18 9671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br><br>Y | WO 2013/036847 A1 (PROLYNX LLC [US]; ASHLEY GARY W [US] ET AL.)<br>14 March 2013 (2013-03-14)<br>* paragraphs [0009] - [0011], [0051], [0054] - [0062], [0092] *<br>* paragraphs [0026], [0084], [0085], [0088], [0089] *<br>* examples 32,33 *<br>----- | 12<br><br><br>1-11, 13-15 | INV.<br>A61K47/69<br>A61K47/60<br>C07K14/605<br>A61K38/00<br>A61P3/10<br>A61K38/26 |
| X<br><br><br>Y | ERIC L. SCHNEIDER ET AL: "Hydrogel Drug Delivery System Using Self-Cleaving Covalent Linkers for Once-a-Week Administration of Exenatide",<br>BIOCONJUGATE CHEMISTRY,<br>vol. 27, no. 5, 1 March 2016 (2016-03-01), pages 1210-1215, XP055637686,<br>US<br>ISSN: 1043-1802, DOI: 10.1021/acs.bioconjchem.5b00690<br>* the whole document *<br>----- | 12<br><br><br>1-11, 13-15 | |
| X<br><br><br>Y | WO 2013/059323 A1 (PROLYNX LLC [US]; SCHNEIDER ERIC L [US] ET AL.)<br>25 April 2013 (2013-04-25)<br>* paragraphs [0001], [0011], [0015] - [0018], [0030], [0031] *<br>* examples 5-8 *<br>* figure 3 *<br>----- | 12<br><br><br>3-11,13, 14 | **TECHNICAL FIELDS SEARCHED (IPC)**<br>A61K<br>C07K<br>A61P |
| Y | US 2010/009904 A1 (LV AIFENG [CN] ET AL)<br>14 January 2010 (2010-01-14)<br>* paragraphs [0005], [0018], [0019] *<br>* compound HR49; sequence 52 *<br>* example 2 *<br>-----<br>-/-- | 1-11, 13-15 | |

1

The present search report has been drawn up for all claims

| Place of search | Date of completion of the search | Examiner |
|---|---|---|
| The Hague | 4 November 2021 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 18 9671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2011/306549 A1 (TATARKIEWICZ KRYSTYNA [US] ET AL) 15 December 2011 (2011-12-15)<br>* paragraph [0041] *<br>* sequence 16 *<br>----- | 1-11, 13-15 | |
| Y | US 2006/194719 A1 (EBBEHOJ KIRSTEN [DK] ET AL) 31 August 2006 (2006-08-31)<br>* figure 9; compound 10 *<br>* paragraphs [0001], [0222], [0242] - [0244] *<br>* figure 8 *<br>----- | 13,14 | |
| Y | US 2007/037750 A1 (YOUNG ANDREW A [US] ET AL) 15 February 2007 (2007-02-15)<br>* paragraph [0002] *<br>* sequence 237 *<br>----- | 13,14 | |
| Y | US 2010/240586 A1 (BAO WENCHAO [CN] ET AL) 23 September 2010 (2010-09-23)<br>* sequence 214 *<br>----- | 13,14 | TECHNICAL FIELDS SEARCHED (IPC) |
| A | BIKASH MANANDHAR ET AL: "Glucagon-like Peptide-1 (GLP-1) Analogs: Recent Advances, New Possibilities, and Therapeutic Implications",<br>JOURNAL OF MEDICINAL CHEMISTRY,<br>vol. 58, no. 3,<br>12 February 2015 (2015-02-12), pages 1020-1037, XP055402142,<br>US<br>ISSN: 0022-2623, DOI: 10.1021/jm500810s<br>* figure 2 *<br>-----<br>-/-- | 1,13,14 | |

The present search report has been drawn up for all claims

1

| Place of search | Date of completion of the search | Examiner |
|---|---|---|
| The Hague | 4 November 2021 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 18 9671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GEIGER T ET AL: "Deamidation isomerization and racemization at asparaginyl and aspartyl residues in peptides", JOURNAL OF BIOLOGICAL CHEMISTRY, AMERICAN SOCIETY FOR BIOCHEMISTRY AND MOLECULAR BIOLOGY, US, vol. 262, no. 2, 15 January 1987 (1987-01-15), pages 785-794, XP002966442, ISSN: 0021-9258 * abstract * * figure 1 * ----- | 1,13,14 | |

TECHNICAL FIELDS SEARCHED (IPC)

The present search report has been drawn up for all claims

1

| Place of search | Date of completion of the search | Examiner |
|---|---|---|
| The Hague | 4 November 2021 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 21 18 9671

This annex lists the patent family members relating to the patent documents cited in the above-mentioned European search report. The members are as contained in the European Patent Office EDP file on The European Patent Office is in no way liable for these particulars which are merely given for the purpose of information.

04-11-2021

| Patent document cited in search report | Publication date | Patent family member(s) | Publication date |
|---|---|---|---|
| WO 2013036847 A1 | 14-03-2013 | AU 2012304337 A1 | 24-04-2014 |
| | | BR 112014005390 A2 | 26-01-2021 |
| | | CA 2848142 A1 | 14-03-2013 |
| | | CN 103945870 A | 23-07-2014 |
| | | DK 2755691 T3 | 17-09-2018 |
| | | EP 2755691 A1 | 23-07-2014 |
| | | ES 2686927 T3 | 22-10-2018 |
| | | HK 1199709 A1 | 17-07-2015 |
| | | JP 6158185 B2 | 05-07-2017 |
| | | JP 6895935 B2 | 30-06-2021 |
| | | JP 2014531433 A | 27-11-2014 |
| | | JP 2017214385 A | 07-12-2017 |
| | | JP 2019031498 A | 28-02-2019 |
| | | KR 20140062114 A | 22-05-2014 |
| | | KR 20200051845 A | 13-05-2020 |
| | | MX 358840 B | 05-09-2018 |
| | | NZ 623512 A | 24-06-2016 |
| | | SG 11201400444V A | 28-04-2014 |
| | | US 2014288190 A1 | 25-09-2014 |
| | | US 2017312368 A1 | 02-11-2017 |
| | | US 2019209691 A1 | 11-07-2019 |
| | | US 2019209692 A1 | 11-07-2019 |
| | | US 2020054756 A1 | 20-02-2020 |
| | | WO 2013036847 A1 | 14-03-2013 |
| | | ZA 201401933 B | 26-08-2015 |
| WO 2013059323 A1 | 25-04-2013 | EP 2768856 A1 | 27-08-2014 |
| | | US 2014256626 A1 | 11-09-2014 |
| | | WO 2013059323 A1 | 25-04-2013 |
| US 2010009904 A1 | 14-01-2010 | CA 2603630 A1 | 20-07-2006 |
| | | CN 1976948 A | 06-06-2007 |
| | | EP 1845105 A1 | 17-10-2007 |
| | | EP 2505207 A2 | 03-10-2012 |
| | | ES 2541633 T3 | 22-07-2015 |
| | | HK 1095045 A1 | 20-04-2007 |
| | | JP 4785206 B2 | 05-10-2011 |
| | | JP 5528380 B2 | 25-06-2014 |
| | | JP 2008526899 A | 24-07-2008 |
| | | JP 2011173886 A | 08-09-2011 |
| | | US 2010009904 A1 | 14-01-2010 |
| | | WO 2006074600 A1 | 20-07-2006 |
| US 2011306549 A1 | 15-12-2011 | US 2011306549 A1 | 15-12-2011 |
| | | US 2012295850 A1 | 22-11-2012 |
| | | WO 2011156407 A2 | 15-12-2011 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 21 18 9671

This annex lists the patent family members relating to the patent documents cited in the above-mentioned European search report. The members are as contained in the European Patent Office EDP file on
The European Patent Office is in no way liable for these particulars which are merely given for the purpose of information.

04-11-2021

| Patent document cited in search report | Publication date | Patent family member(s) | Publication date |
|---|---|---|---|
| US 2006194719 A1 | 31-08-2006 | AU 2003268621 A1 | 04-05-2004 |
| | | AU 2009201477 A1 | 07-05-2009 |
| | | BR 0314996 A | 09-08-2005 |
| | | CA 2500295 A1 | 29-04-2004 |
| | | EP 1546200 A2 | 29-06-2005 |
| | | JP 2006517908 A | 03-08-2006 |
| | | KR 20050083713 A | 26-08-2005 |
| | | MX PA05003335 A | 05-07-2005 |
| | | RU 2376314 C2 | 20-12-2009 |
| | | US 2006194719 A1 | 31-08-2006 |
| | | US 2009208565 A1 | 20-08-2009 |
| | | WO 2004035623 A2 | 29-04-2004 |
| | | ZA 200502571 B | 22-02-2006 |
| US 2007037750 A1 | 15-02-2007 | AT 347901 T | 15-01-2007 |
| | | AT 460942 T | 15-04-2010 |
| | | AU 770712 B2 | 26-02-2004 |
| | | AU 2004202293 A1 | 17-06-2004 |
| | | BR 0007823 A | 20-11-2001 |
| | | CA 2356331 A1 | 20-07-2000 |
| | | CN 1347327 A | 01-05-2002 |
| | | DE 60032331 T2 | 21-06-2007 |
| | | DK 1143989 T3 | 16-04-2007 |
| | | EP 1143989 A2 | 17-10-2001 |
| | | EP 1658856 A1 | 24-05-2006 |
| | | ES 2278589 T3 | 16-08-2007 |
| | | ES 2343072 T3 | 22-07-2010 |
| | | HK 1046105 A1 | 27-12-2002 |
| | | JP 2002538084 A | 12-11-2002 |
| | | KR 20020001719 A | 09-01-2002 |
| | | KR 20070051948 A | 18-05-2007 |
| | | NO 328077 B1 | 23-11-2009 |
| | | NO 2010010 I1 | 07-06-2010 |
| | | NO 2010012 I1 | 07-06-2010 |
| | | NZ 512657 A | 30-01-2004 |
| | | PT 1143989 E | 30-03-2007 |
| | | US 6872700 B1 | 29-03-2005 |
| | | US 2005171019 A1 | 04-08-2005 |
| | | US 2007037750 A1 | 15-02-2007 |
| | | WO 0041548 A2 | 20-07-2000 |
| US 2010240586 A1 | 23-09-2010 | AU 2007321649 A1 | 22-05-2008 |
| | | BR PI0717183 A2 | 16-06-2015 |
| | | CA 2666182 A1 | 22-05-2008 |
| | | CA 2933795 A1 | 22-05-2008 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 21 18 9671

This annex lists the patent family members relating to the patent documents cited in the above-mentioned European search report. The members are as contained in the European Patent Office EDP file on
The European Patent Office is in no way liable for these particulars which are merely given for the purpose of information.

04-11-2021

| Patent document cited in search report | Publication date | Patent family member(s) | Publication date |
|---|---|---|---|
| | | CN 101125207 A | 20-02-2008 |
| | | CN 102827284 A | 19-12-2012 |
| | | EP 2081957 A1 | 29-07-2009 |
| | | JP 2010509248 A | 25-03-2010 |
| | | JP 2014088384 A | 15-05-2014 |
| | | KR 20090089316 A | 21-08-2009 |
| | | RU 2009122582 A | 20-12-2010 |
| | | US 2010240586 A1 | 23-09-2010 |
| | | WO 2008058461 A1 | 22-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8680315 B
- US 8754190 B
- US 8703907 B
- US 8946405 B
- US 20140288190 A
- US 20060194719 A
- WO 2008116294 A, Matregen
- WO 2013036847 A
- WO 2006136586 A


### Non-patent literature cited in the description


- **CAI et al.** *Drug Design, Development, and Therapy,* 2013, vol. 7, 963-970
- **GEIGER ; CLARKE.** *J. Biol. Chem.,* 1987, vol. 262, 785-794
- **ROBINSON ; ROBINSON.** *Proc Natl Acad Sci,* 2010, vol. 98, 12409-12413
- **ZHANG et al.** *Nature Commun.,* 2015, vol. 6, 8918
- **REID, R. et al.** *Macromolecules,* 2015, vol. 48, 7359-7369
- **SCHNEIDER, E. L. et al.** *Biocong. Chem,* 01 March 2016

SEQUENCE LISTING

<110> PROLYNX LLC

<120> EXTENDED RELEASE CONJUGATES OF EXENATIDE ANALOGS

<130> P120749EP10

<140> PCT/US2017/022791
<141> 2017-03-16

<150> US 62/416,058
<151> 2016-11-01

<150> US 62/309,330
<151> 2016-03-16

<160> 12

<170> FastSEQ for Windows Version 4.0

<210> 1
<211> 39
<212> PRT
<213> Artificial Sequence

<220>
<223> Synthetic

<221> AMIDATION
<222> 39
<223> modified to have NH2

<400> 1
```
His Gly Glu Gly Thr Phe Thr Ser Asp Leu Ser Lys Gln Met Glu Glu
 1               5                  10                  15
Glu Ala Val Arg Leu Phe Ile Glu Trp Leu Lys Asn Gly Gly Pro Ser
            20                  25                  30
Ser Gly Ala Pro Pro Pro Ser
        35
```

<210> 2
<211> 39
<212> PRT
<213> Artificial Sequence

<220>
<223> Synthetic

<221> AMIDATION
<222> 39
<223> modified to have NH2

<400> 2
```
His Gly Glu Gly Thr Phe Thr Ser Asp Leu Ser Lys Gln Met Glu Glu
 1               5                  10                  15
Glu Ala Val Arg Leu Phe Ile Glu Trp Leu Lys Gln Gly Gly Pro Ser
            20                  25                  30
Ser Gly Ala Pro Pro Pro Ser
        35
```

```
<210> 3
<211> 39
<212> PRT
<213> Artificial Sequence

<220>
<223> Synthetic

<221> AMIDATION
<222> 39
<223> modified to have NH2

<400> 3
His Gly Glu Gly Thr Phe Thr Ser Asp Leu Ser Lys Gln Met Glu Glu
 1               5                   10                  15
Glu Ala Val Arg Leu Phe Ile Glu Trp Leu Lys Ala Gly Gly Pro Ser
            20                  25                  30
Ser Gly Ala Pro Pro Pro Ser
        35


<210> 4
<211> 39
<212> PRT
<213> Artificial Sequence

<220>
<223> Synthetic

<221> AMIDATION
<222> 39
<223> modified to have NH2

<400> 4
His Gly Glu Gly Thr Phe Thr Ser Asp Leu Ser Lys Gln Met Glu Glu
 1               5                   10                  15
Glu Ala Val Arg Leu Phe Ile Glu Trp Leu Lys Lys Gly Gly Pro Ser
            20                  25                  30
Ser Gly Ala Pro Pro Pro Ser
        35


<210> 5
<211> 39
<212> PRT
<213> Artificial Sequence

<220>
<223> Synthetic

<221> AMIDATION
<222> 39
<223> modified to have NH2

<400> 5
His Gly Glu Gly Thr Phe Thr Ser Asp Leu Ser Lys Gln Met Glu Glu
 1               5                   10                  15
Glu Ala Val Arg Leu Phe Ile Glu Trp Leu Lys Asp Gly Gly Pro Ser
            20                  25                  30
Ser Gly Ala Pro Pro Pro Ser
        35
```

<210> 6
<211> 44
<212> PRT
<213> Artificial Sequence

<220>
<223> Synthetic

<221> AMIDATION
<222> 44
<223> modified to have NH2

<400> 6
His Gly Glu Gly Thr Phe Thr Ser Asp Leu Ser Lys Gln Met Glu Glu
1 5 10 15
Glu Ala Val Arg Leu Phe Ile Glu Trp Leu Lys Asn Gly Gly Pro Ser
20 25 30
Ser Gly Ala Pro Pro Ser Lys Lys Lys Lys Lys Lys
35 40

<210> 7
<211> 44
<212> PRT
<213> Artificial Sequence

<220>
<223> Synthetic

<221> AMIDATION
<222> 44
<223> modified to have NH2

<400> 7
His Gly Glu Gly Thr Phe Thr Ser Asp Leu Ser Lys Gln Met Glu Glu
1 5 10 15
Glu Ala Val Arg Leu Phe Ile Glu Trp Leu Lys Gln Gly Gly Pro Ser
20 25 30
Ser Gly Ala Pro Pro Ser Lys Lys Lys Lys Lys Lys
35 40

<210> 8
<211> 44
<212> PRT
<213> Artificial Sequence

<220>
<223> Synthetic

<221> AMIDATION
<222> 44
<223> modified to have NH2

<400> 8
His Gly Glu Gly Thr Phe Thr Ser Asp Leu Ser Lys Gln Met Glu Glu
1 5 10 15
Glu Ala Val Arg Leu Phe Ile Glu Trp Leu Lys Ala Gly Gly Pro Ser
20 25 30
Ser Gly Ala Pro Pro Ser Lys Lys Lys Lys Lys Lys
35 40

<210> 9
<211> 44
<212> PRT
<213> Artificial Sequence

<220>
<223> Synthetic

<221> AMIDATION
<222> 44
<223> modified to have NH2

<400> 9
His Gly Glu Gly Thr Phe Thr Ser Asp Leu Ser Lys Gln Met Glu Glu
1 5 10 15
Glu Ala Val Arg Leu Phe Ile Glu Trp Leu Lys Lys Gly Gly Pro Ser
20 25 30
Ser Gly Ala Pro Pro Ser Lys Lys Lys Lys Lys Lys
35 40

<210> 10
<211> 40
<212> PRT
<213> Artificial Sequence

<220>
<223> Synthetic

<221> AMIDATION
<222> 40
<223> modified to have NH2

<400> 10
Glu Leu Val Asp Asn Ala Val Gly Gly Asp Leu Ser Lys Gln Met Glu
1 5 10 15
Glu Glu Ala Val Arg Leu Phe Ile Glu Trp Leu Lys Gln Gly Gly Pro
20 25 30
Ser Ser Gly Ala Pro Pro Pro Ser
35 40

<210> 11
<211> 30
<212> PRT
<213> Artificial Sequence

<220>
<223> Synthetic

<220>
<221> AMIDATION
<222> 30
<223> modified to have NH2

<220>
<221> VARIANT
<222> 2, 29
<223> Xaa = Aib

<400> 11

His Xaa Glu Gly Thr Phe Thr Ser Asp Val Ser Ser Tyr Leu Glu Gly
1 5 10 15
Gln Ala Ala Lys Glu Phe Ile Ala Trp Leu Val Lys Xaa Arg
20 25 30

<210> 12
<211> 31
<212> PRT
<213> Artificial Sequence

<220>
<223> Synthetic

<400> 12
His Ala Glu Gly Thr Phe Thr Ser Asp Val Ser Ser Tyr Leu Glu Gly
1 5 10 15
Gln Ala Ala Lys Glu Phe Ile Ala Trp Leu Val Arg Gly Arg Gly
20 25 30